(19) Europäisches Patentamt
European Patent Office
Office européen des brevets

(11) **EP 4 721 766 A1**

(12) **EUROPEAN PATENT APPLICATION**
published in accordance with Art. 153(4) EPC

(43) Date of publication:
**08.04.2026 Bulletin 2026/15**

(21) Application number: **24815603.6**

(22) Date of filing: **31.05.2024**

(51) International Patent Classification (IPC):
***A61K 47/68*** $^{(2017.01)}$ ***A61K 31/416*** $^{(2006.01)}$
***A61K 31/519*** $^{(2006.01)}$ ***A61K 31/4745*** $^{(2006.01)}$
***A61K 39/395*** $^{(2006.01)}$ ***A61K 45/00*** $^{(2006.01)}$
***A61K 47/65*** $^{(2017.01)}$ ***A61P 35/00*** $^{(2006.01)}$
***A61P 43/00*** $^{(2006.01)}$ ***C07K 16/28*** $^{(2006.01)}$

(52) Cooperative Patent Classification (CPC):
**A61K 31/416; A61K 31/4745; A61K 31/519; A61K 39/395; A61K 45/00; A61K 47/65; A61K 47/68; A61P 35/00; A61P 43/00; C07K 16/28**

(86) International application number:
**PCT/JP2024/020005**

(87) International publication number:
**WO 2024/248123 (05.12.2024 Gazette 2024/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.06.2023 JP 2023091440**

(71) Applicant: **Daiichi Sankyo Company, Limited**
**Tokyo 103-8426 (JP)**

(72) Inventors:
- **SUZUKI Hirokazu**
  **Tokyo 103-8426 (JP)**
- **YASUI Rei**
  **Tokyo 103-8426 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

# (54) COMBINATION OF (ANTI-HER3 ANTIBODY)-DRUG CONJUGATE AND RASG12C INHIBITOR

(57) The present invention relates a pharmaceutical product comprising an anti-HER3 antibody-drug conjugate and a RASG12C inhibitor, and/or a method of treatment characterized by administering an anti-HER-3 antibody-drug conjugate and a RASG12C inhibitor in combination to a subject.

Fig. 3

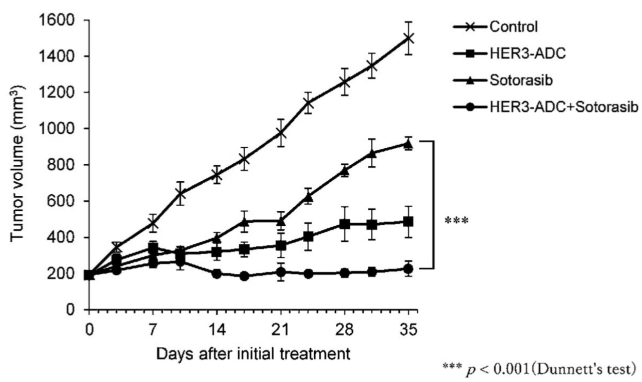


*** $p < 0.001$ (Dunnett's test)



EP 4 721 766 A1

## Description

[Technical Field]

The present invention relates to a pharmaceutical product comprising an anti-HER3 antibody-drug conjugate and a RASG12C inhibitor, and/or a method of treatment characterized by administering an anti-HER3 antibody-drug conjugate and a RASG12C inhibitor in combination to a subject.

[Background Art]

Ras proteins are an important component of signaling pathways that direct growth, differentiation, proliferation, and survival of cells. RAS genes are oncogenes highly frequently mutated in human cancer, and about 30% of all human cancers have mutations in KRAS, NRAS, or HRAS gene. Oncogenic Ras is associated with a mutation in glycine 12, glycine 13, or glutamine 61 of Ras. These residues are positioned in an active site of Ras, and the mutation causes abnormal activation of a downstream effector pathway (MAPK or PI3K pathway). KRAS is a RAS gene most frequently mutated in cancers of some tumor types having a high frequency of activating mutations in KRAS, including pancreatic cancer (prevalence of about 90%); colorectal cancer (prevalence of about 40%); and non-small-cell lung cancer (prevalence of about 30%). KRAS mutations may, in some cases, occur in other cancer types including multiple myeloma, uterine cancer, bile duct cancer, stomach cancer, bladder cancer, diffuse large B-cell lymphoma, rhabdomyosarcoma, skin squamous cell cancer, cervical cancer, and testicular germ cell cancer (Patent Literature 1).

G12C mutation generally occurs in RAS genes, and accounts for 14% of all KRAS mutations, 2% of all NRAS mutations, and 2% of all HRAS mutations of various cancer types. G12C mutation occurs particularly in many cases of KRAS mutant non-small-cell lung cancer, and about a half of cases of the cancer have this mutation. Not only lung cancer is associated with G12C mutation, as G12C mutation occurs also in other RAS-mutated cancer types including in 8% of all KRAS mutant colorectal cancers (Patent Literature 1).

Examples of known compounds having inhibitory activity against G12C mutant Ras protein (RASG12C inhibitors) include LY3537982, AZD4625, sotorasib (AMG510), adagrasib (MRTX849), JDQ443, GDC-6036, BI1, 823, 911, D1553, and JNJ-74699157.

Human epidermal growth factor receptor 3 (also known as HER3, and ErbB3) is a transmembrane receptor belonging to an epidermal growth factor receptor subfamily of receptor protein tyrosine kinase. It is known that an increase in the expression level of HER3 in cancer cells is relevant to the acquisition of resistance to EGFR-TKI (Non-Patent Literature 1). Furthermore, a study has been conducted in order to verify the effect of an anti-HER3 antibody against non-small-cell lung cancer (Non-Patent Literature 2).

Antibody-drug conjugates (ADCs) in which a cytotoxic drug is conjugated to an antibody that binds to an antigen that is expressed on a cancer cell surface and which can be internalized into the cell, can selectively deliver the drug to cancer cells, and hence it is expected that the drug can be accumulated in the cancer cells to kill the cancer cells (Non-Patent Literatures 3 to 7).

As one such antibody-drug conjugate, an antibody-drug conjugate comprising, as components, an anti-HER3 antibody and exatecan, a topoisomerase I inhibitor, is known (Patent Literature 2).

On the other hand, test results showing combined effects of the anti-HER3 antibody-drug conjugate and a RASG12C inhibitor, and a scientific basis suggesting such test results have not been known.

[Citation List]

[Patent Literature]

[Patent Literature 1] International Publication No. WO2020/165732
[Patent Literature 2] International Publication No. WO2015/155998

[Non-Patent Literature]

[Non-Patent Literature 1] N.V. Sergina et al., Nature 2007 January 25; 445(7126): 437-441
[Non-Patent Literature 2] K Yonesaka et al., Oncogene (2016) 35, 878-886.
[Non-Patent Literature 3] Ducry, L., et al., Bioconjugate Chem. (2010) 21, 5-13.

[Non-Patent Literature 4] Alley, S. C., et al., Current Opinion in Chemical Biology (2010) 14, 529-537.
[Non-Patent Literature 5] Damle N. K. Expert Opin. Biol. Ther. (2004) 4, 1445-1452.
[Non-Patent Literature 6] Senter P. D., et al., Nature Biotechnology (2012) 30, 631-637.
[Non-Patent Literature 7] Howard A. et al., J Clin Oncol 29: 398-405.

[Summary of Invention]

[Technical Problem]

It has been confirmed that an anti-HER3 antibody-drug conjugate (an antibody-drug conjugate comprising a derivative of exatecan as a component) used in the present invention exhibits an excellent antitumor effect, even when used alone. When it is used in combination with another anticancer drug having a different mechanism of action, however, a treatment method capable of multiply inhibiting proliferation of cancer cells, and exhibiting a more excellent antitumor effect is desired to be obtained.

An object of the present invention is to provide a pharmaceutical product characterized in that an anti-HER3 antibody-drug conjugate and a RASG12C inhibitor are to be administered in combination, and/or a method of treatment characterized in that an anti-HER3 antibody-drug conjugate and a RASG12C inhibitor are administered in combination to a subject.

[Solution to Problem]

The present inventors have made earnest studies to solve the above-described problems, and as a result, have found that an excellent combined effect is exhibited when an anti-HER3 antibody-drug conjugate and a RASG12C inhibitor are administered in combination, and thus, the present invention has been accomplished.

Specifically, in various embodiments, the present invention provides the following:

[A1]
A pharmaceutical product comprising an anti-HER3 antibody-drug conjugate and a RASG12C inhibitor.
[A2]
The pharmaceutical product according to [A1], wherein the anti-HER3 antibody-drug conjugate is an anti-HER3 antibody-drug conjugate in which a drug-linker represented by the following formula:

[Formula 1]

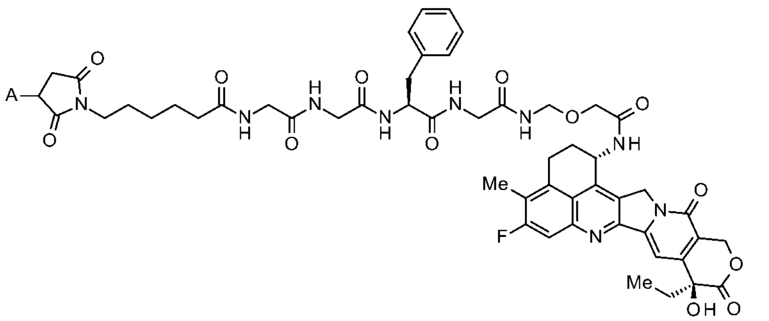

wherein A represents a connecting position to an anti-HER3 antibody, is conjugated to the anti-HER3 antibody via a thioether bond.
[A3]
The pharmaceutical product according to [A1] or [A2], wherein the anti-HER3 antibody is an antibody comprising: a heavy chain comprising CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and a light chain comprising CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.
[A4]
The pharmaceutical product according to [A1] or [A2], wherein the anti-HER3 antibody is an antibody comprising a

heavy chain comprising a heavy chain variable region consisting of an amino acid sequence of amino acid residues 1 to 117 in SEQ ID NO: 1, and a light chain comprising a light chain variable region consisting of an amino acid sequence of amino acid residues 1 to 113 in SEQ ID NO: 2.

[A5]

The pharmaceutical product according to [A1] or [A2], wherein the anti-HER3 antibody is an antibody comprising a heavy chain consisting of an amino acid sequence represented by SEQ ID NO: 1, and a light chain consisting of an amino acid sequence represented by SEQ ID NO: 2.

[A6]

The pharmaceutical product according to [A5], wherein the anti-HER3 antibody lacks a lysine residue at the carboxyl terminus of the heavy chain.

[A7]

The pharmaceutical product according to any one of [A1] to [A6], wherein the average number of units of the drug-linker conjugated per antibody molecule in the anti-HER3 antibody-drug conjugate is in the range of 7 to 8.

[A8]

The pharmaceutical product according to any one of [A1] to [A6], wherein the average number of units of the drug-linker conjugated per antibody molecule in the anti-HER3 antibody-drug conjugate is in the range of 7.5 to 8.

[A9]

The pharmaceutical product according to any one of [A1] to [A8], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

[A10]

The pharmaceutical product according to any one of [A1] to [A8], wherein the RASG12C inhibitor is sotorasib (AMG510), adagrasib (MRTX849), or a pharmaceutically acceptable salt thereof.

[A11]

The pharmaceutical product according to any one of [A1] to [A8], wherein the RASG12C inhibitor is sotorasib (AMG510), or a pharmaceutically acceptable salt thereof.

[A12]

The pharmaceutical product according to any one of [A1] to [A8], wherein the RASG12C inhibitor is adagrasib (MRTX849), or a pharmaceutically acceptable salt thereof.

[A13]

The pharmaceutical product according to any one of [A1] to [A12], wherein the antibody-drug conjugate and the RASG12C inhibitor are comprised in different formulations as active ingredients, and are to be administered at the same time or at different times.

[A14]

The pharmaceutical product according to any one of [A1] to [A13], for use in treating cancer.

[A15]

The pharmaceutical product according to any one of [A1] to [A14], for use in treating at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.

[A16]

The pharmaceutical product according to [A15], for use in treating breast cancer.

[A17]

The pharmaceutical product according to [A15], for use in treating stomach cancer.

[A18]

The pharmaceutical product according to [A15], for use in treating lung cancer.

[A19]

The pharmaceutical product according to [A18], wherein the lung cancer is non-small-cell lung cancer.

[A20]

The pharmaceutical product according to any one of [A1] to [A19], wherein the pharmaceutical product is a pharmaceutical composition.

[B1]

An anti-HER3 antibody-drug conjugate, for use in combination with a RASG12C inhibitor, in treating cancer.

[B2]

The antibody-drug conjugate according to [B1], wherein the anti-HER3 antibody-drug conjugate is an anti-HER3 antibody-drug conjugate in which a drug-linker represented by the following formula:

[Formula 2]

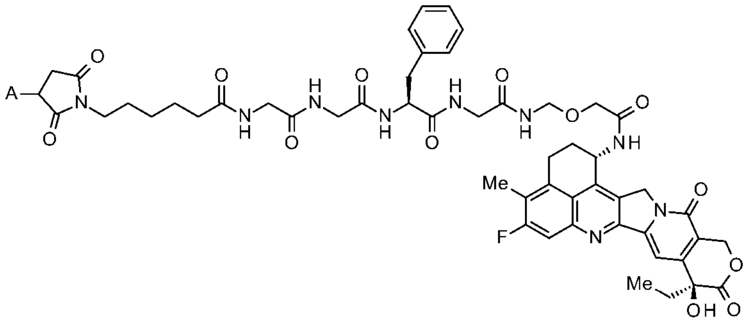

wherein A represents a connecting position to an anti-HER3 antibody,
is conjugated to the anti-HER3 antibody via a thioether bond.

[B3]

The antibody-drug conjugate according to [B1] or [B2], wherein the anti-HER3 antibody is an antibody comprising: a heavy chain comprising CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and a light chain comprising CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.

[B4]

The antibody-drug conjugate according to [B1] or [B2], wherein the anti-HER3 antibody is an antibody comprising a heavy chain comprising a heavy chain variable region consisting of an amino acid sequence of amino acid residues 1 to 117 in SEQ ID NO: 1, and a light chain comprising a light chain variable region consisting of an amino acid sequence of amino acid residues 1 to 113 in SEQ ID NO: 2.

[B5]

The antibody-drug conjugate according to [B1] or [B2], wherein the anti-HER3 antibody is an antibody comprising a heavy chain consisting of an amino acid sequence represented by SEQ ID NO: 1, and a light chain consisting of an amino acid sequence represented by SEQ ID NO: 2.

[B6]

The antibody-drug conjugate according to [B5], wherein the anti-HER3 antibody lacks a lysine residue at the carboxyl terminus of the heavy chain.

[B7]

The antibody-drug conjugate according to any one of [B1] to [B6], wherein the average number of units of the drug-linker conjugated per antibody molecule in the anti-HER3 antibody-drug conjugate is in the range of 7 to 8.

[B8]

The antibody-drug conjugate according to any one of [B1] to [B6], wherein the average number of units of the drug-linker conjugated per antibody molecule in the anti-HER3 antibody-drug conjugate is in the range of 7.5 to 8.

[B9]

The antibody-drug conjugate according to any one of [B1] to [B8], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

[B10]

The antibody-drug conjugate according to any one of [B1] to [B8], wherein the RASG12C inhibitor is sotorasib (AMG510), adagrasib (MRTX849), or a pharmaceutically acceptable salt thereof.

[B11]

The antibody-drug conjugate according to any one of [B1] to [B8], wherein the RASG12C inhibitor is sotorasib (AMG510), or a pharmaceutically acceptable salt thereof.

[B12]

The antibody-drug conjugate according to any one of [B1] to [B8], wherein the RASG12C inhibitor is adagrasib (MRTX849), or a pharmaceutically acceptable salt thereof.

[B13]

The antibody-drug conjugate according to any one of [B1] to [B12], wherein the antibody-drug conjugate and the RASG12C inhibitor are comprised in different formulations as active ingredients, and are to be administered at the

same time or at different times.
[B14]
The antibody-drug conjugate according to any one of [B1] to [B13], wherein the cancer is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.
[B15]
The antibody-drug conjugate according to [B14], wherein the cancer is breast cancer.
[B16]
The antibody-drug conjugate according to [B14], wherein the cancer is stomach cancer.
[B17]
The antibody-drug conjugate according to [B14], wherein the cancer is lung cancer.
[B18]
The antibody-drug conjugate according to [B17], wherein the lung cancer is non-small-cell lung cancer.
[C1]
A RASG12C inhibitor, for use in combination with an anti-HER3 antibody-drug conjugate, in treating cancer.
[C2]
The inhibitor according to [C1], wherein the anti-HER3 antibody-drug conjugate is an anti-HER3 antibody-drug conjugate in which a drug-linker represented by the following formula:

[Formula 3]

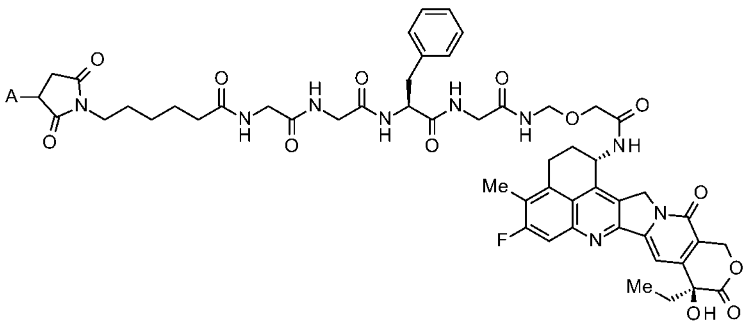

wherein A represents a connecting position to an anti-HER3 antibody,
is conjugated to the anti-HER3 antibody via a thioether bond.
[C3]
The inhibitor according to [C1] or [C2], wherein the anti-HER3 antibody is an antibody comprising: a heavy chain comprising CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and a light chain comprising CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.
[C4]
The inhibitor according to [C1] or [C2], wherein the anti-HER3 antibody is an antibody comprising a heavy chain comprising a heavy chain variable region consisting of an amino acid sequence of amino acid residues 1 to 117 in SEQ ID NO: 1, and a light chain comprising a light chain variable region consisting of an amino acid sequence of amino acid residues 1 to 113 in SEQ ID NO: 2.
[C5]
The inhibitor according to [C1] or [C2], wherein the anti-HER3 antibody is an antibody comprising a heavy chain consisting of an amino acid sequence represented by SEQ ID NO: 1, and a light chain consisting of an amino acid sequence represented by SEQ ID NO: 2.
[C6]
The inhibitor according to [C5], wherein the anti-HER3 antibody lacks a lysine residue at the carboxyl terminus of the heavy chain.
[C7]

The inhibitor according to any one of [C1] to [C6], wherein the average number of units of the drug-linker conjugated per antibody molecule in the anti-HER3 antibody-drug conjugate is in the range of 7 to 8.

[C8]

The inhibitor according to any one of [C1] to [C6], wherein the average number of units of the drug-linker conjugated per antibody molecule in the anti-HER3 antibody-drug conjugate is in the range of 7.5 to 8.

[C9]

The inhibitor according to any one of [C1] to [C8], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

[C10]

The inhibitor according to any one of [C1] to [C8], wherein the RASG12C inhibitor is sotorasib (AMG510), adagrasib (MRTX849), or a pharmaceutically acceptable salt thereof.

[C11]

The inhibitor according to any one of [C1] to [C8], wherein the RASG12C inhibitor is sotorasib (AMG510), or a pharmaceutically acceptable salt thereof.

[C12]

The inhibitor according to any one of [C1] to [C8], wherein the RASG12C inhibitor is adagrasib (MRTX849), or a pharmaceutically acceptable salt thereof.

[C13]

The inhibitor according to any one of [C1] to [C12], wherein the antibody-drug conjugate and the RASG12C inhibitor are comprised in different formulations as active ingredients, and are administered at the same time or at different times.

[C14]

The inhibitor according to any one of [C1] to [C13], wherein the cancer is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.

[C15]

The inhibitor according to [C14], wherein the cancer is breast cancer.

[C16]

The inhibitor according to [C14], wherein the cancer is stomach cancer.

[C17]

The inhibitor according to [C14], wherein the cancer is lung cancer.

[C18]

The inhibitor according to [C17], wherein the lung cancer is non-small-cell lung cancer.

[D1]

Use of an anti-HER3 antibody-drug conjugate in combination with a RASG12C inhibitor in the manufacture of a medicament for treating cancer.

[D2]

The use according to [D1], wherein the anti-HER3 antibody-drug conjugate is an anti-HER3 antibody-drug conjugate in which a drug-linker represented by the following formula:

[Formula 4]

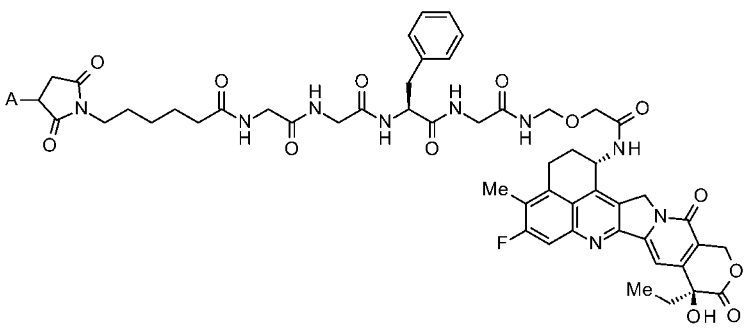

wherein A represents a connecting position to an anti-HER3 antibody,

is conjugated to the anti-HER3 antibody via a thioether bond.

[D3]

The use according to [D1] or [D2], wherein the anti-HER3 antibody is an antibody comprising: a heavy chain comprising CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and a light chain comprising CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.

[D4]

The use according to [D1] or [D2], wherein the anti-HER3 antibody is an antibody comprising a heavy chain comprising a heavy chain variable region consisting of an amino acid sequence of amino acid residues 1 to 117 in SEQ ID NO: 1, and a light chain comprising a light chain variable region consisting of an amino acid sequence of amino acid residues 1 to 113 in SEQ ID NO: 2.

[D5]

The use according to [D1] or [D2], wherein the anti-HER3 antibody is an antibody comprising a heavy chain consisting of an amino acid sequence represented by SEQ ID NO: 1, and a light chain consisting of an amino acid sequence represented by SEQ ID NO: 2.

[D6]

The use according to [D5], wherein the anti-HER3 antibody lacks a lysine residue at the carboxyl terminus of the heavy chain.

[D7]

The use according to any one of [D1] to [D6], wherein the average number of units of the drug-linker conjugated per antibody molecule in the anti-HER3 antibody-drug conjugate is in the range of 7 to 8.

[D8]

The use according to any one of [D1] to [D6], wherein the average number of units of the drug-linker conjugated per antibody molecule in the anti-HER3 antibody-drug conjugate is in the range of 7.5 to 8.

[D9]

The use according to any one of [D1] to [D8], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

[D10]

The use according to any one of [D1] to [D8], wherein the RASG12C inhibitor is sotorasib (AMG510), adagrasib (MRTX849), or a pharmaceutically acceptable salt thereof.

[D11]

The use according to any one of [D1] to [D8], wherein the RASG12C inhibitor is sotorasib (AMG510), or a pharmaceutically acceptable salt thereof.

[D12]

The use according to any one of [D1] to [D8], wherein the RASG12C inhibitor is adagrasib (MRTX849), or a pharmaceutically acceptable salt thereof.

[D13]

The use according to any one of [D1] to [D12], wherein the antibody-drug conjugate and the RASG12C inhibitor are comprised in different formulations as active ingredients, and are administered at the same time or at different times.

[D14]

The use according to any one of [D1] to [D13], wherein the cancer is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.

[D15]

The use according to [D14], wherein the cancer is breast cancer.

[D16]

The use according to [D14], wherein the cancer is stomach cancer.

[D17]

The use according to [D14], wherein the cancer is lung cancer.

[D18]

The use according to [D17], wherein the lung cancer is non-small-cell lung cancer.

[E1]

A method of treatment for a disease, comprising administering an anti-HER3 antibody-drug conjugate and a RASG12C inhibitor in combination to a subject in need of treatment.

[E2]

The method of treatment according to [E1], wherein the anti-HER3 antibody-drug conjugate is an anti-HER3 antibody-drug conjugate in which a drug-linker represented by the following formula:

[Formula 5]

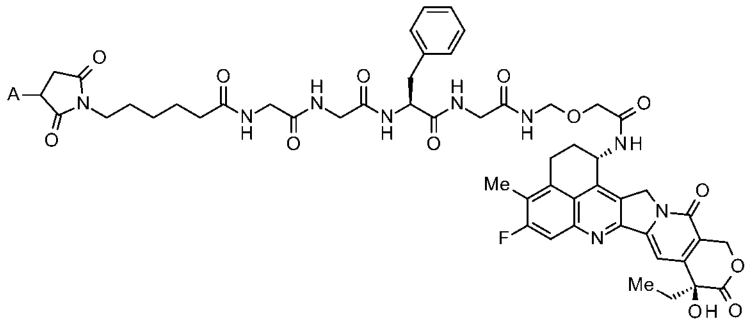

wherein A represents a connecting position to an anti-HER3 antibody,
is conjugated to the anti-HER3 antibody via a thioether bond.

[E3]

The method of treatment according to [E1] or [D2], wherein the anti-HER3 antibody is an antibody comprising: a heavy chain comprising CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and a light chain comprising CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.

[E4]

The method of treatment according to [E1] or [E2], wherein the anti-HER3 antibody is an antibody comprising a heavy chain comprising a heavy chain variable region consisting of an amino acid sequence of amino acid residues 1 to 117 in SEQ ID NO: 1, and a light chain comprising a light chain variable region consisting of an amino acid sequence of amino acid residues 1 to 113 in SEQ ID NO: 2.

[E5]

The method of treatment according to [E1] or [E2], wherein the anti-HER3 antibody is an antibody comprising a heavy chain consisting of an amino acid sequence represented by SEQ ID NO: 1, and a light chain consisting of an amino acid sequence represented by SEQ ID NO: 2.

[E6]

The method of treatment according to [D5], wherein the anti-HER3 antibody lacks a lysine residue at the carboxyl terminus of the heavy chain.

[E7]

The method of treatment according to any one of [E1] to [E6], wherein the average number of units of the drug-linker conjugated per antibody molecule in the anti-HER3 antibody-drug conjugate is in the range of 7 to 8.

[E8]

The method of treatment according to any one of [E1] to [E6], wherein the average number of units of the drug-linker conjugated per antibody molecule in the anti-HER3 antibody-drug conjugate is in the range of 7.5 to 8.

[E9]

The method of treatment according to any one of [E1] to [E8], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

[E10]

The method of treatment according to any one of [E1] to [E8], wherein the RASG12C inhibitor is sotorasib (AMG510), adagrasib (MRTX849), or a pharmaceutically acceptable salt thereof.

[E11]

The method of treatment according to any one of [E1] to [E8], wherein the RASG12C inhibitor is sotorasib (AMG510), or a pharmaceutically acceptable salt thereof.

[E12]
The method of treatment according to any one of [E1] to [E8], wherein the RASG12C inhibitor is adagrasib (MRTX849), or a pharmaceutically acceptable salt thereof.
[E13]
The method of treatment according to any one of [E1] to [E12], wherein the antibody-drug conjugate and the RASG12C inhibitor are comprised in different formulations as active ingredients, and are administered at the same time or at different times.
[E14]
The method of treatment according to any one of [E1] to [E13], wherein the disease is cancer.
[E15]
The method of treatment according to any one of [E1] to [E14], wherein the disease is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.
[E16]
The method of treatment according to [E15], wherein the disease is breast cancer.
[E17]
The method of treatment according to [E15], wherein the disease is stomach cancer.
[E18]
The method of treatment according to [E15], wherein the disease is lung cancer.
[E19]
The method of treatment according to [E18], wherein the lung cancer is non-small-cell lung cancer.
[A1-1]
A pharmaceutical product comprising an anti-HER3 antibody-drug conjugate and a RASG12C inhibitor.
[A2-1]
The pharmaceutical product according to [A1-1], wherein the anti-HER3 antibody-drug conjugate is an anti-HER3 antibody-drug conjugate in which a drug-linker represented by the following formula:

[Formula 6]

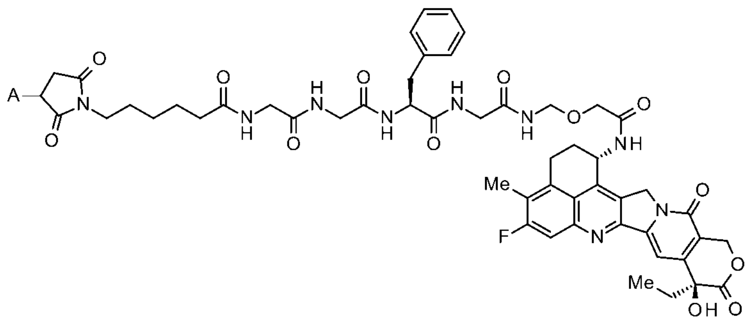

wherein A represents a connecting position to an anti-HER3 antibody or a functional fragment thereof,
is conjugated to the anti-HER3 antibody or functional fragment thereof via a thioether bond.
[A3-1]
The pharmaceutical product according to [A1-1] or [A2-1], wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.
[A4-1]
The pharmaceutical product according to [A1-1] or [A2-1], wherein the anti-HER3 antibody or functional fragment thereof comprises a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.

[A5-1]
The pharmaceutical product according to [A1-1] or [A2-1], wherein the anti-HER3 antibody or functional fragment thereof comprises a heavy chain consisting of an amino acid sequence represented by SEQ ID NO: 1, and a light chain consisting of an amino acid sequence represented by SEQ ID NO: 2.
[A6-1]
The pharmaceutical product according to [A5-1], wherein the anti-HER3 antibody lacks a lysine residue at the carboxyl terminus of the heavy chain.
[A7-1]
The pharmaceutical product according to any one of [A1-1] to [A6-1], wherein the average number of units of the drug-linker conjugated per antibody molecule in the anti-HER3 antibody-drug conjugate is in the range of 2 to 8.
[A8-1]
The pharmaceutical product according to any one of [A1-1] to [A6-1], wherein the average number of units of the drug-linker conjugated per antibody molecule in the anti-HER3 antibody-drug conjugate is in the range of 3 to 8.
[A9-1]
The pharmaceutical product according to any one of [A1-1] to [A6-1], wherein the average number of units of the drug-linker conjugated per antibody molecule in the anti-HER3 antibody-drug conjugate is in the range of 7 to 8.
[A10-1]
The pharmaceutical product according to any one of [A1-1] to [A6-1], wherein the average number of units of the drug-linker conjugated per antibody molecule in the anti-HER3 antibody-drug conjugate is in the range of 7.5 to 8.
[A11-1]
The pharmaceutical product according to any one of [A1-1] to [A6-1], wherein the average number of units of the drug-linker conjugated per antibody molecule in the anti-HER3 antibody-drug conjugate is 8.
[A12-1]
The pharmaceutical product according to any one of [A1-1] to [A11-1], wherein the anti-HER3 antibody-drug conjugate is patritumab deruxtecan.
[A13-1]
The pharmaceutical product according to any one of [A1-1] to [A12-1], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.
[A14-1]
The pharmaceutical product according to any one of [A1-1] to [A12-1], wherein the RASG12C inhibitor is sotorasib (AMG510), adagrasib (MRTX849), or a pharmaceutically acceptable salt thereof.
[A15-1]
The pharmaceutical product according to any one of [A1-1] to [A12-1], wherein the RASG12C inhibitor is sotorasib (AMG510), or a pharmaceutically acceptable salt thereof.
[A16-1]
The pharmaceutical product according to any one of [A1-1] to [A12-1], wherein the RASG12C inhibitor is adagrasib (MRTX849), or a pharmaceutically acceptable salt thereof.
[A17-1]
The pharmaceutical product according to any one of [A1-1] to [A16-1], wherein the antibody-drug conjugate and the RASG12C inhibitor are comprised in different formulations (as active ingredients), and are to be administered at the same time or at different times.
[A18-1]
The pharmaceutical product according to any one of [A1-1] to [A17-1], for use in treating cancer.
[A19-1]
The pharmaceutical product according to any one of [A1-1] to [A18-1], for use in treating at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.
[A20-1]
The pharmaceutical product according to [A19-1], for use in treating breast cancer.
[A21-1]
The pharmaceutical product according to [A19-1], for use in treating stomach cancer.
[A22-1]
The pharmaceutical product according to [A19-1], for use in treating lung cancer.
[A23-1]
The pharmaceutical product according to [A22-1], wherein the lung cancer is non-small-cell lung cancer.

[A24-1]
The pharmaceutical product according to any one of [A1-1] to [A23-1], wherein the pharmaceutical product is a pharmaceutical composition.
[B1-1]
An anti-HER3 antibody-drug conjugate, for use in combination with a RASG12C inhibitor, in treating cancer.
[B2-1]
The antibody-drug conjugate according to [B1-1], wherein the anti-HER3 antibody-drug conjugate or functional fragment thereof is an anti-HER3 antibody-drug conjugate in which a drug-linker represented by the following formula:

[Formula 7]

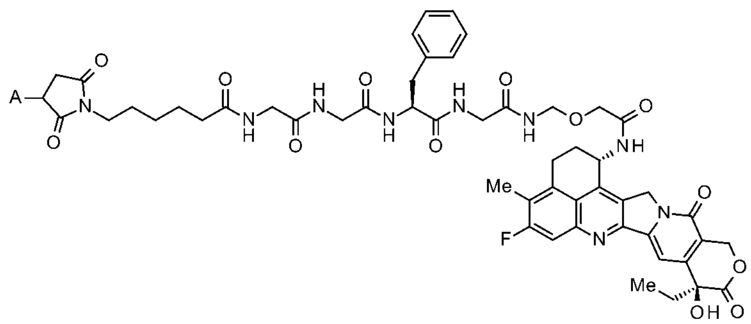

wherein A represents a connecting position to an anti-HER3 antibody,
is conjugated to the anti-HER3 antibody or functional fragment thereof via a thioether bond.
[B3-1]
The antibody-drug conjugate according to [B1-1] or [B2-1], wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.
[B4-1]
The antibody-drug conjugate according to [B1-1] or [B2-1], wherein the anti-HER3 antibody or functional fragment thereof comprises a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.
[B5-1]
The antibody-drug conjugate according to [B1-1] or [B2-1], wherein the anti-HER3 antibody or functional fragment thereof comprises a heavy chain consisting of an amino acid sequence represented by SEQ ID NO: 1, and a light chain consisting of an amino acid sequence represented by SEQ ID NO: 2.
[B6-1]
The antibody-drug conjugate according to [B5-1], wherein the anti-HER3 antibody lacks a lysine residue at the carboxyl terminus of the heavy chain.
[B7-1]
The antibody-drug conjugate according to any one of [B1-1] to [B6-1], wherein the average number of units of the drug-linker conjugated per antibody molecule in the anti-HER3 antibody-drug conjugate is in the range of 2 to 8.
[B8-1]
The antibody-drug conjugate according to any one of [B1-1] to [B6-1], wherein the average number of units of the drug-linker conjugated per antibody molecule in the anti-HER3 antibody-drug conjugate is in the range of 3 to 8.
[B9-1]
The antibody-drug conjugate according to any one of [B1-1] to [B6-1], wherein the average number of units of the drug-linker conjugated per antibody molecule in the anti-HER3 antibody-drug conjugate is in the range of 7 to 8.
[B10-1]
The antibody-drug conjugate according to any one of [B1-1] to [B6-1], wherein the average number of units of the drug-linker conjugated per antibody molecule in the anti-HER3 antibody-drug conjugate is in the range of 7.5 to 8.
[B11-1]

The antibody-drug conjugate according to any one of [B1-1] to [B6-1], wherein the average number of units of the drug-linker conjugated per antibody molecule in the anti-HER3 antibody-drug conjugate is 8.

[B12-1]

The antibody-drug conjugate according to any one of [B1-1] to [B11-1], wherein the anti-HER3 antibody-drug conjugate is patritumab deruxtecan.

[B13-1]

The antibody-drug conjugate according to any one of [B1-1] to [B12-1], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

[B14-1]

The antibody-drug conjugate according to any one of [B1-1] to [B12-1], wherein the RASG12C inhibitor is sotorasib (AMG510), adagrasib (MRTX849), or a pharmaceutically acceptable salt thereof.

[B15-1]

The antibody-drug conjugate according to any one of [B1-1] to [B12-1], wherein the RASG12C inhibitor is sotorasib (AMG510), or a pharmaceutically acceptable salt thereof.

[B16-1]

The antibody-drug conjugate according to any one of [B1-1] to [B12-1], wherein the RASG12C inhibitor is adagrasib (MRTX849), or a pharmaceutically acceptable salt thereof.

[B17-1]

The antibody-drug conjugate according to any one of [B1-1] to [B16-1], wherein the antibody-drug conjugate and the RASG12C inhibitor are comprised in different formulations (as active ingredients), and are to be administered at the same time or at different times.

[B18-1]

The antibody-drug conjugate according to any one of [B1-1] to [B17-1], wherein the cancer is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.

[B19-1]

The antibody-drug conjugate according to [B18-1], wherein the cancer is breast cancer.

[B20-1]

The antibody-drug conjugate according to [B18-1], wherein the cancer is stomach cancer.

[B21-1]

The antibody-drug conjugate according to [B18-1], wherein the cancer is lung cancer.

[B22-1]

The antibody-drug conjugate according to [B21-1], wherein the lung cancer is non-small-cell lung cancer.

[C1-1]

A RASG12C inhibitor, for use in combination with an anti-HER3 antibody-drug conjugate, in treating cancer.

[C2-1]

The inhibitor according to [C1-1], wherein the anti-HER3 antibody-drug conjugate is an anti-HER3 antibody-drug conjugate in which a drug-linker represented by the following formula:

[Formula 8]

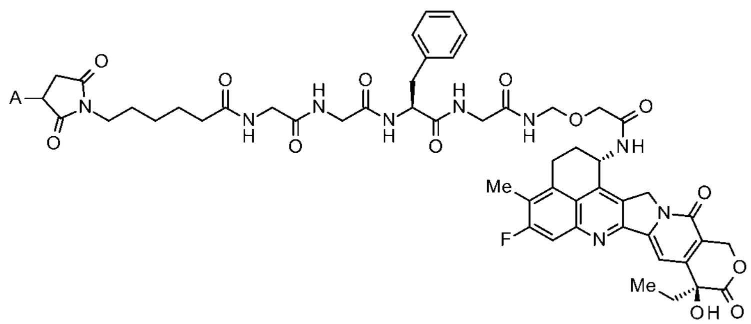

wherein A represents a connecting position to an anti-HER3 antibody or a functional fragment thereof, is conjugated to the anti-HER3 antibody or functional fragment thereof via a thioether bond.

[C3-1]
The inhibitor according to [C1-1] or [C2-1], wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.
[C4-1]
The inhibitor according to [C1-1] or [C2-1], wherein the anti-HER3 antibody or functional fragment thereof comprises a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.
[C5-1]
The inhibitor according to [C1-1] or [C2-1], wherein the anti-HER3 antibody or functional fragment thereof comprises a heavy chain consisting of an amino acid sequence represented by SEQ ID NO: 1, and a light chain consisting of an amino acid sequence represented by SEQ ID NO: 2.
[C6-1]
The inhibitor according to [C5-1], wherein the anti-HER3 antibody lacks a lysine residue at the carboxyl terminus of the heavy chain.
[C7-1]
The inhibitor according to any one of [C1-1] to [C6-1], wherein the average number of units of the drug-linker conjugated per antibody molecule in the anti-HER3 antibody-drug conjugate is in the range of 2 to 8.
[C8-1]
The inhibitor according to any one of [C1-1] to [C6-1], wherein the average number of units of the drug-linker conjugated per antibody molecule in the anti-HER3 antibody-drug conjugate is in the range of 3 to 8.
[C9-1]
The inhibitor according to any one of [C1-1] to [C6-1], wherein the average number of units of the drug-linker conjugated per antibody molecule in the anti-HER3 antibody-drug conjugate is in the range of 7 to 8.
[C10-1]
The inhibitor according to any one of [C1-1] to [C6-1], wherein the average number of units of the drug-linker conjugated per antibody molecule in the anti-HER3 antibody-drug conjugate is in the range of 7.5 to 8.
[C11-1]
The inhibitor according to any one of [C1-1] to [C6-1], wherein the average number of units of the drug-linker conjugated per antibody molecule in the anti-HER3 antibody-drug conjugate is 8.
[C12-1]
The inhibitor according to any one of [C1-1] to [C11-1], wherein the anti-HER3 antibody-drug conjugate is patritumab deruxtecan.
[C13-1]
The inhibitor according to any one of [C1-1] to [C12-1], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.
[C14-1]
The inhibitor according to any one of [C1-1] to [C12-1], wherein the RASG12C inhibitor is sotorasib (AMG510), adagrasib (MRTX849), or a pharmaceutically acceptable salt thereof.
[C15-1]
The inhibitor according to any one of [C1-1] to [C12-1], wherein the RASG12C inhibitor is sotorasib (AMG510), or a pharmaceutically acceptable salt thereof.
[C16-1]
The inhibitor according to any one of [C1-1] to [C12-1], wherein the RASG12C inhibitor is adagrasib (MRTX849), or a pharmaceutically acceptable salt thereof.
[C17-1]
The inhibitor according to any one of [C1-1] to [C16-1], wherein the antibody-drug conjugate and the RASG12C inhibitor are comprised in different formulations (as active ingredients), and are administered at the same time or at different times.
[C18-1]
The inhibitor according to any one of [C1-1] to [C17-1], wherein the cancer is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian

cancer, bladder cancer, prostate cancer, uterine carcinosarcoma, gastrointestinal stromal tumor, kidney cancer, and sarcoma.

[C19-1]

The inhibitor according to [C18-1], wherein the cancer is breast cancer.

[C20-1]

The inhibitor according to [C18-1], wherein the cancer is stomach cancer.

[C21-1]

The inhibitor according to [C18-1], wherein the cancer is lung cancer.

[C22-1]

The inhibitor according to [C21-1], wherein the lung cancer is non-small-cell lung cancer.

[D1-1]

Use of an anti-HER3 antibody-drug conjugate in combination with a RASG12C inhibitor in the manufacture of a medicament for treating cancer.

[D2-1]

The use according to [D1-1], wherein the anti-HER3 antibody-drug conjugate is an anti-HER3 antibody-drug conjugate in which a drug-linker represented by the following formula:

[Formula 9]

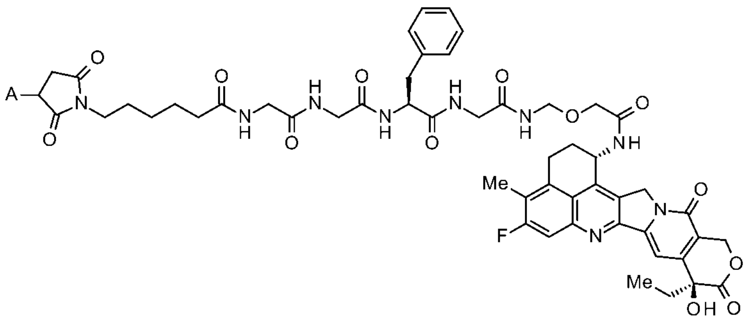

wherein A represents a connecting position to an anti-HER3 antibody or a functional fragment thereof, is conjugated to the anti-HER3 antibody or functional fragment thereof via a thioether bond.

[D3-1]

The use according to [D1-1] or [D2-1], wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.

[D4-1]

The use according to [D1-1] or [D2-1], wherein the anti-HER3 antibody or functional fragment thereof comprises a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.

[D5-1]

The use according to [D1-1] or [D2-1], wherein the anti-HER3 or functional fragment thereof antibody comprises a heavy chain consisting of an amino acid sequence represented by SEQ ID NO: 1, and a light chain consisting of an amino acid sequence represented by SEQ ID NO: 2.

[D6-1]

The use according to [D5-1], wherein the anti-HER3 antibody lacks a lysine residue at the carboxyl terminus of the heavy chain.

[D7-1]

The use according to any one of [D1-1] to [D6-1], wherein the average number of units of the drug-linker conjugated per antibody molecule in the anti-HER3 antibody-drug conjugate is in the range of 2 to 8.

[D8-1]

The use according to any one of [D1-1] to [D6-1], wherein the average number of units of the drug-linker conjugated per antibody molecule in the anti-HER3 antibody-drug conjugate is in the range of 3 to 8.

[D9-1]
The use according to any one of [D1-1] to [D6-1], wherein the average number of units of the drug-linker conjugated per antibody molecule in the anti-HER3 antibody-drug conjugate is in the range of 7 to 8.
[D10-1]
The use according to any one of [D1-1] to [D6-1], wherein the average number of units of the drug-linker conjugated per antibody molecule in the anti-HER3 antibody-drug conjugate is in the range of 7.5 to 8.
[D11-1]
The use according to any one of [D1-1] to [D6-1], wherein the average number of units of the drug-linker conjugated per antibody molecule in the anti-HER3 antibody-drug conjugate is 8.
[D12-1]
The use according to any one of [D1-1] to [D11-1], wherein the anti-HER3 antibody-drug conjugate is patritumab deruxtecan.
[D13-1]
The use according to any one of [D1-1] to [D12-1], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.
[D14-1]
The use according to any one of [D1-1] to [D12-1], wherein the RASG12C inhibitor is sotorasib (AMG510), adagrasib (MRTX849), or a pharmaceutically acceptable salt thereof.
[D15-1]
The use according to any one of [D1-1] to [D12-1], wherein the RASG12C inhibitor is sotorasib (AMG510), or a pharmaceutically acceptable salt thereof.
[D16-1]
The use according to any one of [D1-1] to [D12-1], wherein the RASG12C inhibitor is adagrasib (MRTX849), or a pharmaceutically acceptable salt thereof.
[D17-1]
The use according to any one of [D1-1] to [D16-1], wherein the antibody-drug conjugate and the RASG12C inhibitor are comprised in different formulations (as active ingredients), and are administered at the same time or at different times.
[D18-1]
The use according to any one of [D1-1] to [D17-1], wherein the cancer is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.
[D19-1]
The use according to [D18-1], wherein the cancer is breast cancer.
[D20-1]
The use according to [D18-1], wherein the cancer is stomach cancer.
[D21-1]
The use according to [D18-1], wherein the cancer is lung cancer.
[D22-1]
The use according to [D21-1], wherein the lung cancer is non-small-cell lung cancer.
[E1-1]
A method of treatment for a disease, comprising administering an anti-HER3 antibody-drug conjugate and a RASG12C inhibitor in combination to a subject in need of treatment.
[E2-1]
The method of treatment according to [E1-1], wherein the anti-HER3 antibody-drug conjugate is an anti-HER3 antibody-drug conjugate in which a drug-linker represented by the following formula:

[Formula 10]

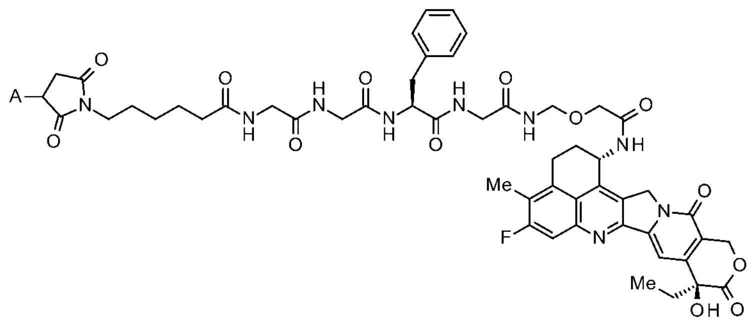

wherein A represents a connecting position to an anti-HER3 antibody or a functional fragment thereof, is conjugated to the anti-HER3 antibody or functional fragment thereof via a thioether bond.

[E3-1]

The method of treatment according to [E1-1] or [E2-1], wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.

[E4-1]

The method of treatment according to [E1-1] or [E2-1], wherein the anti-HER3 antibody or functional fragment thereof is an antibody comprising a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.

[E5-1]

The method of treatment according to [E1-1] or [E2-1], wherein the anti-HER3 antibody comprises a heavy chain consisting of an amino acid sequence represented by SEQ ID NO: 1, and a light chain consisting of an amino acid sequence represented by SEQ ID NO: 2.

[E6-1]

The method of treatment according to [E5-1], wherein the anti-HER3 antibody lacks a lysine residue at the carboxyl terminus of the heavy chain.

[E7-1]

The method of treatment according to any one of [E1-1] to [E6-1], wherein the average number of units of the drug-linker conjugated per antibody molecule in the anti-HER3 antibody-drug conjugate is in the range of 2 to 8.

[E8-1]

The method of treatment according to any one of [E1-1] to [E6-1], wherein the average number of units of the drug-linker conjugated per antibody molecule in the anti-HER3 antibody-drug conjugate is in the range of 3 to 8.

[E9-1]

The method of treatment according to any one of [E1-1] to [E6-1], wherein the average number of units of the drug-linker conjugated per antibody molecule in the anti-HER3 antibody-drug conjugate is in the range of 7 to 8.

[E10-1]

The method of treatment according to any one of [E1-1] to [E6-1], wherein the average number of units of the drug-linker conjugated per antibody molecule in the anti-HER3 antibody-drug conjugate is in the range of 7.5 to 8.

[E11-1]

The method of treatment according to any one of [E1-1] to [E6-1], wherein the average number of units of the drug-linker conjugated per antibody molecule in the anti-HER3 antibody-drug conjugate is 8.

[E12-1]

The method of treatment according to any one of [E1-1] to [E11-1], wherein the anti-HER3 antibody-drug conjugate is patritumab deruxtecan.

[E13-1]

The method of treatment according to any one of [E1-1] to [E12-1], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

[E14-1]
The method of treatment according to any one of [E1-1] to [E12-1], wherein the RASG12C inhibitor is sotorasib (AMG510), adagrasib (MRTX849), or a pharmaceutically acceptable salt thereof.
[E15-1]
The method of treatment according to any one of [E1-1] to [E12-1], wherein the RASG12C inhibitor is sotorasib (AMG510), or a pharmaceutically acceptable salt thereof.
[E16-1]
The method of treatment according to any one of [E1-1] to [E12-1], wherein the RASG12C inhibitor is adagrasib (MRTX849), or a pharmaceutically acceptable salt thereof.
[E17-1]
The method of treatment according to any one of [E1-1] to [E16-1], wherein the antibody-drug conjugate and the RASG12C inhibitor are comprised in different formulations (as active ingredients), and are administered at the same time or at different times.
[E18-1]
The method of treatment according to any one of [E1-1] to [E17-1], wherein the disease is cancer.
[E19-1]
The method of treatment according to any one of [E1-1] to [E18-1], wherein the disease is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.
[E20-1]
The method of treatment according to [E19-1], wherein the disease is breast cancer.
[E21-1]
The method of treatment according to [E19-1], wherein the disease is stomach cancer.
[E22-1]
The method of treatment according to [E19-1], wherein the disease is lung cancer.
[E23-1]
The method of treatment according to [E22-1], wherein the lung cancer is non-small-cell lung cancer.
[A1-2]
A pharmaceutical product comprising an anti-HER3 antibody-drug conjugate and a RASG12C inhibitor.
[A2-2]
The pharmaceutical product according to [Al-2], wherein the anti-HER3 antibody-drug conjugate is represented by the following formula:

[Formula 11]

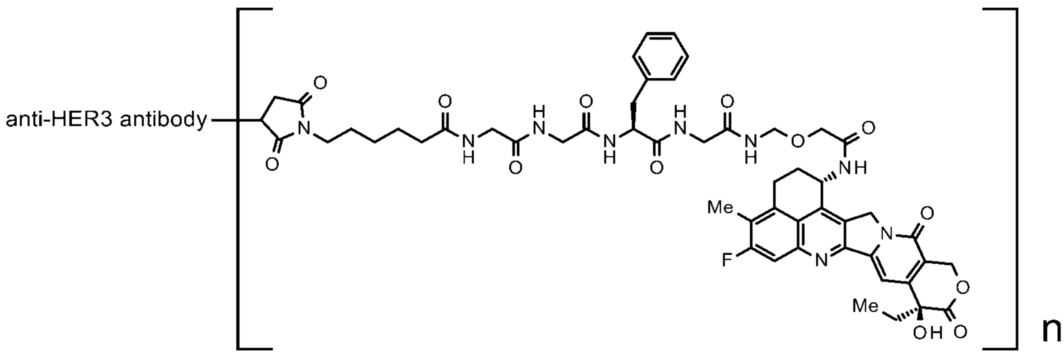

wherein 'anti-HER3 antibody' in the formula is an anti-HER3 antibody or a functional fragment thereof; a drug linker is conjugated to the antibody or functional fragment thereof via a thioether bond; and n represents drug-to-antibody ratio.
[A3-2]
The pharmaceutical product according to [Al-2] or [A2-2], wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino

acid residues 56 to 62 in SEQ ID NO: 2, and CDRL3 consisting of an amino acid sequence of amino acid residues 95 to 103 in SEQ ID NO: 2.

[A4-2]

The pharmaceutical product according to [A1-2] or [A2-2], wherein the anti-HER3 antibody or functional fragment thereof comprises a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.

[A5-2]

The pharmaceutical product according to [Al-2] or [A2-2], wherein the anti-HER3 antibody or functional fragment thereof comprises a heavy chain consisting of an amino acid sequence represented by SEQ ID NO: 1, and a light chain consisting of an amino acid sequence represented by SEQ ID NO: 2.

[A6-2]

The pharmaceutical product according to [A5-2], wherein the anti-HER3 antibody lacks a lysine residue at the carboxyl terminus of the heavy chain.

[A7-2]

The pharmaceutical product according to any one of [Al-2] to [A6-2], wherein n is 2 to 8.

[A8-2]

The pharmaceutical product according to any one of [Al-2] to [A6-2], wherein n is 3 to 8.

[A9-2]

The pharmaceutical product according to any one of [Al-2] to [A6-2], wherein n is 7 to 8.

[A10-2]

The pharmaceutical product according to any one of [A1-2] to [A6-2], wherein n is 7.5 to 8.

[A11-2]

The pharmaceutical product according to any one of [Al-2] to [A6-2], wherein n is 8.

[A12-2]

The pharmaceutical product according to any one of [A1-2] to [A11-2], wherein the anti-HER3 antibody-drug conjugate is patritumab deruxtecan.

[A13-2]

The pharmaceutical product according to any one of [Al-2] to [A12-2], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

[A14-2]

The pharmaceutical product according to any one of [Al-2] to [A12-2], wherein the RASG12C inhibitor is sotorasib (AMG510), adagrasib (MRTX849), or a pharmaceutically acceptable salt thereof.

[A15-2]

The pharmaceutical product according to any one of [Al-2] to [A12-2], wherein the RASG12C inhibitor is sotorasib (AMG510), or a pharmaceutically acceptable salt thereof.

[A16-2]

The pharmaceutical product according to any one of [Al-2] to [A12-2], wherein the RASG12C inhibitor is adagrasib (MRTX849), or a pharmaceutically acceptable salt thereof.

[A17-2]

The pharmaceutical product according to any one of [Al-2] to [A16-2], wherein the antibody-drug conjugate and the RASG12C inhibitor are comprised in different formulations (as active ingredients), and are to be administered at the same time or at different times.

[A18-2]

The pharmaceutical product according to any one of [A1-2] to [A17-2], for use in treating cancer.

[A19-2]

The pharmaceutical product according to any one of [A1-2] to [A18-2], for use in treating at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.

[A20-2]

The pharmaceutical product according to [A19-2], for use in treating breast cancer.

[A21-2]

The pharmaceutical product according to [A19-2], for use in treating stomach cancer.

[A22-2]

The pharmaceutical product according to [A19-2], for use in treating lung cancer.

[A23-2]
The pharmaceutical product according to [A22-2], wherein the lung cancer is non-small-cell lung cancer.
[A24-2]
The pharmaceutical product according to any one of [A1-2] to [A23-2], wherein the pharmaceutical product is a pharmaceutical composition.
[B1-2]
An anti-HER3 antibody-drug conjugate, for use in combination with a RASG12C inhibitor, in treating cancer.
[B2-2]
The antibody-drug conjugate according to [B1-2], wherein the anti-HER3 antibody-drug conjugate or functional fragment thereof is represented by the following formula:

[Formula 12]

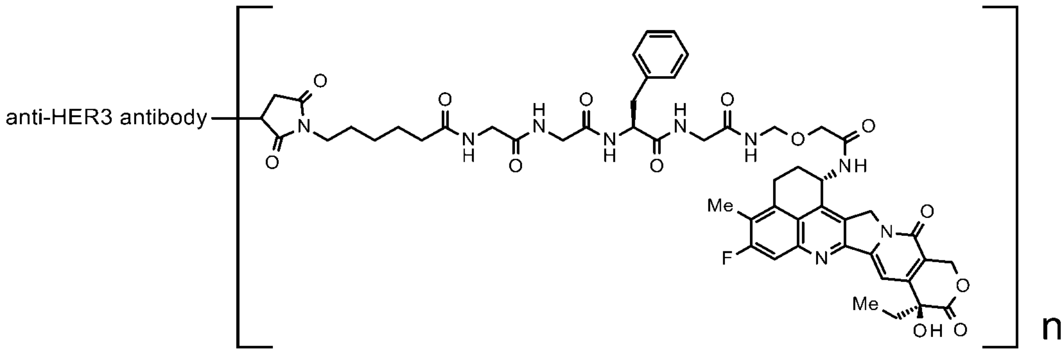

wherein 'anti-HER3 antibody' in the formula is an anti-HER3 antibody or a functional fragment thereof; a drug linker is conjugated to the antibody or functional fragment thereof via a thioether bond; and n represents drug-to-antibody ratio.
[B3-2]
The antibody-drug conjugate according to [B1-2] or [B2-2], wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.
[B4-2]
The antibody-drug conjugate according to [B1-2] or [B2-2], wherein the anti-HER3 antibody or functional fragment thereof comprises a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.
[B5-2]
The antibody-drug conjugate according to [B1-2] or [B2-2], wherein the anti-HER3 antibody or functional fragment thereof comprises a heavy chain consisting of an amino acid sequence represented by SEQ ID NO: 1, and a light chain consisting of an amino acid sequence represented by SEQ ID NO: 2.
[B6-2]
The antibody-drug conjugate according to [B5-2], wherein the anti-HER3 antibody lacks a lysine residue at the carboxyl terminus of the heavy chain.
[B7-2]
The antibody-drug conjugate according to any one of [B1-2] to [B6-2], wherein n is 2 to 8.
[B8-2]
The antibody-drug conjugate according to any one of [B1-2] to [B6-2], wherein n is 3 to 8.
[B9-2]
The antibody-drug conjugate according to any one of [B1-2] to [B6-2], wherein n is 7 to 8.
[B10-2]
The antibody-drug conjugate according to any one of [B1-2] to [B6-2], wherein n is 7.5 to 8.
[B11-2]
The antibody-drug conjugate according to any one of [B1-2] to [B6-2], wherein n is 8.

[B12-2]
The antibody-drug conjugate according to any one of [B1-2] to [B11-2], wherein the anti-HER3 antibody-drug conjugate is patritumab deruxtecan.
[B13-2]
The antibody-drug conjugate according to any one of [B1-2] to [B12-2], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.
[B14-2]
The antibody-drug conjugate according to any one of [B1-2] to [B12-2], wherein the RASG12C inhibitor is sotorasib (AMG510), adagrasib (MRTX849), or a pharmaceutically acceptable salt thereof.
[B15-2]
The antibody-drug conjugate according to any one of [B1-2] to [B12-2], wherein the RASG12C inhibitor is sotorasib (AMG510), or a pharmaceutically acceptable salt thereof.
[B16-2]
The antibody-drug conjugate according to any one of [B1-2] to [B12-2], wherein the RASG12C inhibitor is adagrasib (MRTX849), or a pharmaceutically acceptable salt thereof.
[B17-2]
The antibody-drug conjugate according to any one of [B1-2] to [B17-2], wherein the antibody-drug conjugate and the RASG12C inhibitor are comprised in different formulations (as active ingredients), and are administered at the same time or at different times.
[B18-2]
The antibody-drug conjugate according to any one of [B1-2] to [B17-2], wherein the cancer is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.
[B19-2]
The antibody-drug conjugate according to [B18-2], wherein the cancer is breast cancer.
[B20-2]
The antibody-drug conjugate according to [B18-2], wherein the cancer is stomach cancer.
[B21-2]
The antibody-drug conjugate according to [B18-2], wherein the cancer is lung cancer.
[B22-2]
The antibody-drug conjugate according to [B21-2], wherein the lung cancer is non-small-cell lung cancer.
[C1-2]
A RASG12C inhibitor, for use in combination with an anti-HER3 antibody-drug conjugate, in treating cancer.
[C2-2]
The inhibitor according to [C1-2], wherein the anti-HER3 antibody-drug conjugate is represented by the following formula:

[Formula 13]

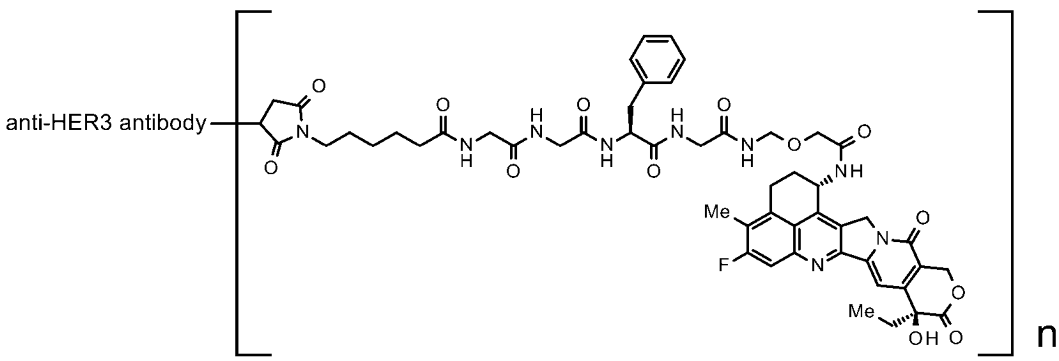

wherein 'anti-HER3 antibody' in the formula is an anti-HER3 antibody or a functional fragment thereof; a drug linker is conjugated to the antibody or functional fragment thereof via a thioether bond; and n represents drug-to-antibody ratio.
[C3-2]

The inhibitor according to [C1-2] or [C2-2], wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.

[C4-2]

The inhibitor according to [C1-2] or [C2-2], wherein the anti-HER3 antibody or functional fragment thereof comprises a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.

[C5-2]

The inhibitor according to [C1-2] or [C2-2], wherein the anti-HER3 antibody or functional fragment thereof comprises a heavy chain consisting of an amino acid sequence represented by SEQ ID NO: 1, and a light chain consisting of an amino acid sequence represented by SEQ ID NO: 2.

[C6-2]

The inhibitor according to [C5-2], wherein the anti-HER3 antibody lacks a lysine residue at the carboxyl terminus of the heavy chain.

[C7-2]

The inhibitor according to any one of [C1-2] to [C6-2], wherein n is 2 to 8.

[C8-2]

The inhibitor according to any one of [C1-2] to [C6-2], wherein n is 3 to 8.

[C9-2]

The inhibitor according to any one of [C1-2] to [C6-2], wherein n is 7 to 8.

[C10-2]

The inhibitor according to any one of [C1-2] to [C6-2], wherein n is 7.5 to 8.

[C11-2]

The inhibitor according to any one of [C1-2] to [C6-2], wherein n is 8.

[C12-2]

The inhibitor according to any one of [C1-2] to [C11-2], wherein the anti-HER3 antibody-drug conjugate is patritumab deruxtecan.

[C13-2]

The inhibitor according to any one of [C1-2] to [C12-2], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

[C14-2]

The inhibitor according to any one of [C1-2] to [C12-2], wherein the RASG12C inhibitor is sotorasib (AMG510), adagrasib (MRTX849), or a pharmaceutically acceptable salt thereof.

[C15-2]

The inhibitor according to any one of [C1-2] to [C12-2], wherein the RASG12C inhibitor is sotorasib (AMG510), or a pharmaceutically acceptable salt thereof.

[C16-2]

The inhibitor according to any one of [C1-2] to [C12-2], wherein the RASG12C inhibitor is adagrasib (MRTX849), or a pharmaceutically acceptable salt thereof.

[C17-2]

The inhibitor according to any one of [C1-2] to [C16-2], wherein the antibody-drug conjugate and the RASG12C inhibitor are comprised in different formulations (as active ingredients), and are administered at the same time or at different times.

[C18-2]

The inhibitor according to any one of [C1-2] to [C17-2], wherein the cancer is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.

[C19-2]

The inhibitor according to [C18-2], wherein the cancer is breast cancer.

[C20-2]

The inhibitor according to [C18-2], wherein the cancer is stomach cancer.

[C21-2]
The inhibitor according to [C18-2], wherein the cancer is lung cancer.
[C22-2]
The inhibitor according to [C21-2], wherein the lung cancer is non-small-cell lung cancer.
[D1-2]
Use of an anti-HER3 antibody-drug conjugate in combination with a RASG12C inhibitor in the manufacture of a medicament for treating cancer.
[D2-2]
The use according to [D1-2], wherein the anti-HER3 antibody-drug conjugate is represented by the following formula:

[Formula 14]

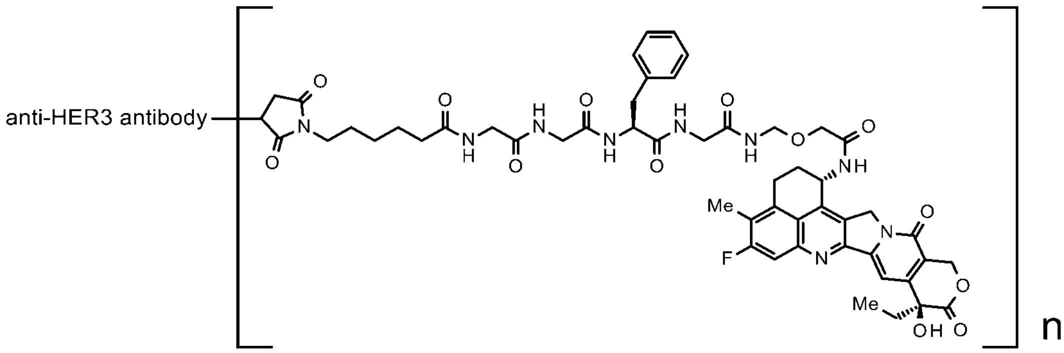

wherein 'anti-HER3 antibody' in the formula is an anti-HER3 antibody or a functional fragment thereof; a drug linker is conjugated to the antibody or functional fragment thereof via a thioether bond; and n represents drug-to-antibody ratio.
[D3-2]
The use according to [D1-2] or [D2-2], wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.
[D4-2]
The use according to [D1-2] or [D2-2], wherein the anti-HER3 antibody or functional fragment thereof comprises a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.
[D5-2]
The use according to [D1-2] or [D2-2], wherein the anti-HER3 antibody or functional fragment thereof comprises a heavy chain consisting of an amino acid sequence represented by SEQ ID NO: 1, and a light chain consisting of an amino acid sequence represented by SEQ ID NO: 2.
[D6-2]
The use according to [D5-2], wherein the anti-HER3 antibody lacks a lysine residue at the carboxyl terminus of the heavy chain.
[D7-2]
The use according to any one of [D1-2] to [D6-2], wherein n is 2 to 8.
[D8-2]
The use according to any one of [D1-2] to [D6-2], wherein n is 3 to 8.
[D9-2]
The use according to any one of [D1-2] to [D6-2], wherein n is 7 to 8.
[D10-2]
The use according to any one of [D1-2] to [D6-2], wherein n is 7.5 to 8.
[D11-2]
The use according to any one of [D1-2] to [D6-2], wherein n is is 8.
[D12-2]
The use according to any one of [D1-2] to [D11-2], wherein the anti-HER3 antibody-drug conjugate is patritumab

deruxtecan.

[D13-2]

The use according to any one of [D1-2] to [D12-2], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

[D14-2]

The use according to any one of [D1-2] to [D12-2], wherein the RASG12C inhibitor is sotorasib (AMG510), adagrasib (MRTX849), or a pharmaceutically acceptable salt thereof.

[D15-2]

The use according to any one of [D1-2] to [D12-2], wherein the RASG12C inhibitor is sotorasib (AMG510), or a pharmaceutically acceptable salt thereof.

[D16-2]

The use according to any one of [D1-2] to [D12-2], wherein the RASG12C inhibitor is adagrasib (MRTX849), or a pharmaceutically acceptable salt thereof.

[D17-2]

The use according to any one of [D1-2] to [D16-2], wherein the antibody-drug conjugate and the RASG12C inhibitor are comprised in different formulations (as active ingredients), and are administered at the same time or at different times.

[D18-2]

The use according to any one of [D1-2] to [D17-2], wherein the cancer is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.

[D19-2]

The use according to [D18-2], wherein the cancer is breast cancer.

[D20-2]

The use according to [D18-2], wherein the cancer is stomach cancer.

[D21-2]

The use according to [D18-2], wherein the cancer is lung cancer.

[D22-2]

The use according to [D21-2], wherein the lung cancer is non-small-cell lung cancer.

[E1-2]

A method of treatment for a disease, comprising administering an anti-HER3 antibody-drug conjugate and a RASG12C inhibitor in combination to a subject in need of treatment.

[E2-2]

The method of treatment according to [E1-2], wherein the anti-HER3 antibody-drug conjugate is represented by the following formula:

[Formula 15]

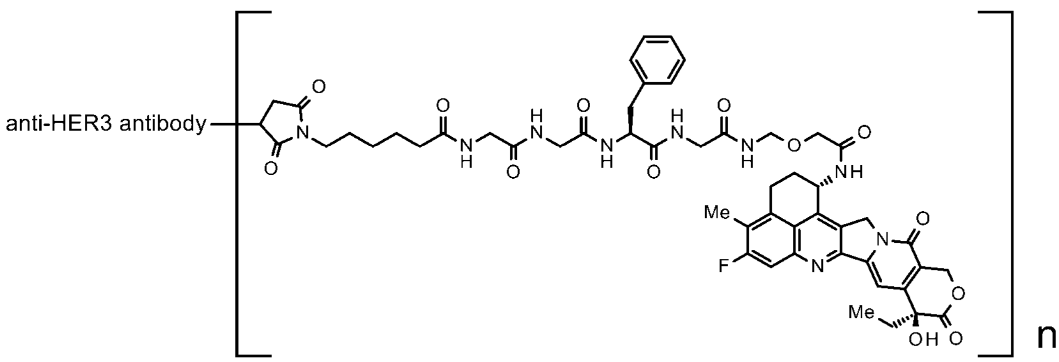

wherein 'anti-HER3 antibody' in the formula is an anti-HER3 antibody or a functional fragment thereof; a drug linker is conjugated to the antibody or functional fragment thereof via a thioether bond; and n represents drug-to-antibody ratio.

[E3-2]

The method of treatment according to [E1-2] or [E2-2], wherein the anti-HER3 antibody or functional fragment thereof

comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.

[E4-2]

The method of treatment according to [E1-2] or [E2-2], wherein the anti-HER3 antibody or functional fragment thereof is an antibody comprising a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.

[E5-2]

The method of treatment according to [E1-2] or [E2-2], wherein the anti-HER3 antibody or functional fragment thereof comprises a heavy chain consisting of an amino acid sequence represented by SEQ ID NO: 1, and a light chain consisting of an amino acid sequence represented by SEQ ID NO: 2.

[E6-2]

The method of treatment according to [E5-2], wherein the anti-HER3 antibody lacks a lysine residue at the carboxyl terminus of the heavy chain.

[E7-2]

The method of treatment according to any one of [E1-2] to [E6-2], wherein n is 2 to 8.

[E8-2]

The method of treatment according to any one of [E1-2] to [E6-2], wherein n is 3 to 8.

[E9-2]

The method of treatment according to any one of [E1-2] to [E6-2], wherein n is 7 to 8.

[E10-2]

The method of treatment according to any one of [E1-2] to [E6-2], wherein n is 7.5 to 8.

[E11-2]

The method of treatment according to any one of [E1-2] to [E6-2], wherein n is 8.

[E12-2]

The method of treatment according to any one of [E1-2] to [E11-2], wherein the anti-HER3 antibody-drug conjugate is patritumab deruxtecan.

[E13-2]

The method of treatment according to any one of [E1-2] to [E12-2], wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

[E14-2]

The method of treatment according to any one of [E1-2] to [E12-2], wherein the RASG12C inhibitor is sotorasib (AMG510), adagrasib (MRTX849), or a pharmaceutically acceptable salt thereof.

[E15-2]

The method of treatment according to any one of [E1-2] to [E12-2], wherein the RASG12C inhibitor is sotorasib (AMG510), or a pharmaceutically acceptable salt thereof.

[E16-2]

The method of treatment according to any one of [E1-2] to [E12-2], wherein the RASG12C inhibitor is adagrasib (MRTX849), or a pharmaceutically acceptable salt thereof.

[E17-2]

The method of treatment according to any one of [E1-2] to [E16-2], wherein the antibody-drug conjugate and the RASG12C inhibitor are comprised in different formulations as active ingredients, and are administered at the same time or at different times.

[E18-2]

The method of treatment according to any one of [E1-2] to [E17-2], wherein the disease is cancer.

[E19-2]

The method of treatment according to any one of [E1-2] to [E18-2], wherein the disease is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.

[E20-2]

The method of treatment according to [E19-2], wherein the disease is breast cancer.

[E21-2]
The method of treatment according to [E19-2], wherein the disease is stomach cancer.
[E22-2]
The method of treatment according to [E19-2], wherein the disease is lung cancer.
[E23-2]
The method of treatment according to [E22-2], wherein the lung cancer is non-small-cell lung cancer.
[F1-1]
A pharmaceutical product comprising (i) an anti-HER3 antibody-drug conjugate and (ii) a RASG12C inhibitor, wherein the anti-HER3 antibody-drug conjugate and the RASG12C inhibitor are to be administered in combination, and the anti-HER3 antibody-drug conjugate and the RASG12C inhibitor are as defined in any one of [A2-1] to [A16-1] and [A2-2] to [A16-2].
[F2-1]
The pharmaceutical product according to [F1-1], for use in treating a disease defined in any one of [A18-1] to [A23-1].
[F3-1]
The pharmaceutical product according to [F1-1] or [F2-1], wherein the antibody-drug conjugate and the RASG12C inhibitor are comprised in different formulations (as active ingredients), and are to be administered at the same time or at different times.
[F4-1]
The pharmaceutical product according to [F1-1] or [F2-1], wherein the antibody-drug conjugate and the RASG12C inhibitor are contained (as active components) in a single formulation for administration.
[F5-1]
The pharmaceutical product according to [F1-1] or [F4-1], wherein the pharmaceutical product is a pharmaceutical composition.
[F6-1]
A kit comprising (i) a first composition comprising an anti-HER3 antibody-drug conjugate and (ii) a second composition comprising a RASG12C inhibitor, wherein the anti-HER3 antibody-drug conjugate and the RASG12C inhibitor are to be administered in combination, and the anti-HER3 antibody-drug conjugate and the RASG12C inhibitor are as defined in any one of [A2-1] to [A16-1] and [A2-2] to [A16-2].
[F7-1]
The kit according to [F6-1], for use in treating a disease defined in any one of [A18-1] to [A23-1].
[F8-1]
The kit according to [F6-1] or [F7-1], wherein the antibody-drug conjugate and the RASG12C inhibitor are comprised in different formulations (as active ingredients), and are to be administered at the same time or at different times.
[F9-1]
The kit according to [F6-1] or [F7-1], wherein the antibody-drug conjugate and the RASG12C inhibitor are contained (as active components) in a single formulation for administration.
[F10-1]
A method of treatment for cancer, comprising administering an anti-HER3 antibody-drug conjugate and a RASG12C inhibitor in combination to a subject in need of treatment, wherein the anti-HER3 antibody-drug conjugate and the RASG12C inhibitor are as defined in any one of [A2-1] to [A16-1] and [A2-2] to [A16-2].
[F11-1]
A pharmaceutical product comprising an anti-HER3 antibody-drug conjugate, wherein the anti-HER3 antibody-drug conjugate is to be administered in combination with a RASG12C inhibitor, and the anti-HER3 antibody-drug conjugate is represented by the following formula:

[Formula 16]

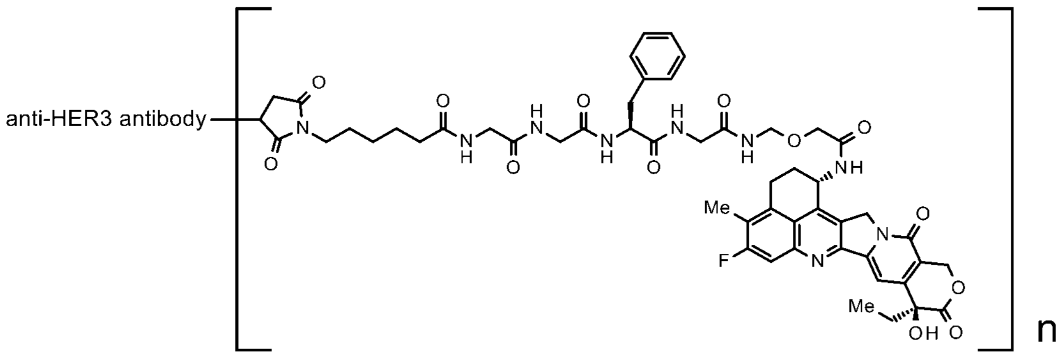

wherein 'anti-HER3 antibody' in the formula is an anti-HER3 antibody or a functional fragment thereof; each drug-linker represented by the structure shown within the bracket in the formula is conjugated to the antibody or functional fragment thereof via a thioether bond; and n represents an average number of units of the drug-linker conjugated per antibody.

[Advantageous Effects of Invention]

The present invention can provide a pharmaceutical product characterized in that an anti-HER3 antibody-drug conjugate and a RASG12C inhibitor are to be administered in combination, and/or a method of treatment characterized in that an anti-HER3 antibody-drug conjugate and a RASG12C inhibitor are administered in combination to a subject.

[Brief Description of Drawings]

[Figure 1] Figure 1 is a diagram illustrating an amino acid sequence of an anti-HER3 antibody heavy chain (SEQ ID NO: 1).
[Figure 2] Figure 2 is a diagram illustrating an amino acid sequence of an anti-HER3 antibody light chain (SEQ ID NO: 2).
[Figure 3] Figure 3 is a diagram illustrating a tumor growth inhibitory effect in groups each given one of HER3-ADC (1) and sotorasib, and in a group given both HER3-ADC (1) and sotorasib in combination, in mice into which NCI-H358 cells have been subcutaneously transplanted.
[Figure 4] Figure 4 is a diagram illustrating a tumor proliferation inhibitory effect in groups each given one of HER3-ADC (1) and adagrasib, and in a group given both HER3-ADC (1) and adagrasib in combination, in mice into which NCI-H358 cells have been subcutaneously transplanted.

[Description of Embodiment]

Now, a preferred embodiment for practicing the present invention will be described. It is noted that the embodiment described below is merely an example of a typical embodiment of the present invention, and that the scope of the present invention is not to be interpreted narrowly by this embodiment.

1. RASG12C Inhibitor

As used herein, the term "RASG12C inhibitor" refers to a compound having inhibitory activity against a G12C mutant Ras protein, a protein encoded by KRAS, NRAS, or HRAS gene, and having G12C mutation in one aspect, and more preferably a protein encoded by KRAS gene and having G12C mutation. Examples include compounds selected from compounds described in detail in International Publication No. WO2013/155223, International Publication No. WO2014/143659, International Publication No. WO2014/152588, International Publication No. WO2014/160200, International Publication No. WO2015/054572, International Publication No. WO2016/044772, International Publication No. WO2016/049524, International Publication No. WO2016/164675, International Publication No. WO2016/168540, International Publication No. WO2017/058805, International Publication No. WO2017/015562, International Publication No.

WO2017/058728, International Publication No. WO2017/058768, International Publication No. WO2017/058792, International Publication No. WO2017/058805, International Publication No. WO2017/058807, International Publication No. WO2017/058902, International Publication No. WO2017/058915, International Publication No. WO2017/087528, International Publication No. WO2017/100546, International Publication No. 2017/201161, International Publication No. 2018/064510, International Publication No. 2018/068017, International Publication No. 2018/119183, International Publication No. 2018/217651, International Publication No. 2018/140512, International Publication No. 2018/140513, International Publication No. 2018/140514, International Publication No. 2018/140598, International Publication No. 2018/140599, International Publication No. 2018/140600, International Publication No. 2018/143315, International Publication No. 2018/206539, International Publication No. 2018/218070, International Publication No. 2018/218071, International Publication No. 2019/051291, International Publication No. 2019/099524, International Publication No. 2019/110751, International Publication No. 2019/141250, International Publication No. 2019/150305, International Publication No. 2019/155399, International Publication No. 2019/213516, International Publication No. 2019/213526, International Publication No. 2019/217307, International Publication No. 2019/215203, International Publication No. 2019/217691, International Publication No. 2020/178282, International Publication No. WO2021/118877, and International Publication No. WO2021/245051.

In another aspect, the "RASG12C inhibitor" can be a compound selected from the group consisting of LY3537982, AZD4625, sotorasib (AMG510), adagrasib (MRTX849), JDQ443, GDC-6036, BI1, 823, 911, D1553, JNJ-74699157, and pharmaceutically acceptable salts thereof.

In another aspect, the "RASG12C inhibitor" can be sotorasib (AMG510), adagrasib (MRTX849), or a pharmaceutically acceptable salt thereof.

In another aspect, the "RASG12C inhibitor" can be sotorasib (AMG510), or a pharmaceutically acceptable salt thereof.

In another aspect, the "RASG12C inhibitor" can be adagrasib (MRTX849), or a pharmaceutically acceptable salt thereof.

Such a RASG12C inhibitor can be produced by a method described in any of the above-described literatures, or a known method.

As used herein, the term "pharmaceutically acceptable salt" refers to a salt that does not have notable toxicity, and that can be used as a medicament.

A compound having an acidic substituent can be formed into a salt by a reaction with a base. Examples include, but are not limited to, alkali metal salts such as sodium salt, potassium salt, and lithium salt; alkaline earth metal salts such as calcium salt, and magnesium salt; metal salts such as aluminum salt, and iron salt; inorganic salts such as ammonium salt; amine salts including organic salts and the like such as tertbutylamine salt, tert-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenetylamine salt, piperazine salt, tetramethylammonium salt, and tris(hydroxymethyl)aminomethane salt; and amino acid salts such as glycine salt, lysine salt, arginine salt, ornithine salt, glutamic acid salt, and aspartic acid salt.

A compound having a basic substituent can be formed into a salt by a reaction with an acid. Examples include halide acid salts such as hydrofluoric acid salt, hydrochloric acid salt, hydrobromic acid salt, and hydroiodic acid salt; inorganic acid salts such as nitric acid salt, perchloric acid salt, sulfuric acid salt, and phosphoric acid salt; C1-C6 alkyl sulfonic acid salts such as methanesulfonic acid salt, trifluoromethanesulfonic acid salt, and ethanesulfonic acid salt; aryl sulfonic acid salts such as benzenesulfonic acid salt, and p-toluenesulfonic acid salt; organic acid salts such as acetic acid salt, malic acid salt, fumaric acid salt, succinic acid salt, citric acid salt, ascorbic acid salt, tartaric acid salt, oxalic acid salt, adipic acid salt, and maleic acid salt; and amino acid salts such as glycine salt, lysine salt, arginine salt, ornithine salt, glutamic acid salt, and aspartic acid salt.

2. Anti-HER3 Antibody

As used herein, the term "HER3" is defined the same as "human epidermal growth factor receptor 3" (also referred to as ErbB3), and is a transmembrane receptor belonging to an epidermal growth factor receptor subfamily of receptor protein tyrosine kinase, together with HER1, HER2, and HER4. HER3 is expressed in some types of cancer such as breast cancer, gastrointestinal cancer, and pancreatic cancer, and is known to selfphosphorylate by forming a heterodimer together with a tyrosine kinase receptor such as EGFR or HER2 to induce proliferation of cancer cells and an apoptosis-inhibitory signal.

The HER3 protein used in the present invention may be directly purified from a human HER3 expressing cell for use, or for use as an antigen, a cell membrane fraction of the cell can be used as the HER3 protein. Alternatively, the HER3 protein can be obtained by synthesizing HER3 *in vitro,* or by causing a host cell to produce it by genetic engineering. In genetic engineering, specifically, HER3 can be synthesized by incorporating a vector capable of expressing HER3 cDNA,

and incubating the resultant vector in a solution containing an enzyme necessary for transcription and translation, a substrate, and an energy substance. Alternatively, the protein can be obtained by transforming another prokaryote, or a host cell of an eukaryote with the vector to express HER3. Furthermore, a HER3 expressing cell, or a cell line expressing HER3 obtained by genetic engineering can be used as a HER3 protein antigen.

The RNA sequence, the cDNA sequence, and the amino acid sequence of HER3 are disclosed in public databases, and are available with accession numbers such as AAA35979 (precursor containing a signal sequence consisting of 19 amino terminal amino acid residues), and M34309 (NCBI).

Furthermore, HER3 also encompasses a protein having an amino acid sequence obtained by substituting, lacking, adding, and/or inserting 1 to 10 amino acids in the amino acid sequence of HER3, and having biological activity equivalent to that of the protein.

As used herein, the term "anti-HER3 antibody" refers to an antibody having activity that causes its specific binding to HER3, and preferably, internalization into a HER3 expressing cell mediated by binding to HER3, in other words, an antibody having activity that causes its translocation into a HER3 expressing cell after binding to HER3.

The anti-HER3 antibody used in the present invention may be derived from any species, and is an antibody preferably derived from a human, a rat, a mouse, or a rabbit. When the antibody is derived from species other than a human, it is preferably chimerized or humanized by a known technique. The antibody of the present invention may be a polyclonal antibody, or a monoclonal antibody, and is preferably a monoclonal antibody.

The HER3 antibody used in the present invention preferably has a property capable of targeting a cancer cell, and those having a property capable of recognizing a cancer cell, a property capable of binding to a cancer cell, a property of being incorporated and internalized into a cancer cell, and/or cancer cell killing activity or the like are preferred.

The binding activity of the anti-HER3 antibody to a cancer cell can be confirmed by employing flow cytometry. The incorporation of the antibody into a cancer cell can be confirmed by (1) assay for visualizing an antibody incorporated into a cell by using a secondary antibody (fluorescent label) that binds to a therapeutic antibody (Cell Death and Differentiation (2008) 15, 751-761), (2) assay for measuring the amount of fluorescence incorporated into a cell by using a secondary antibody (fluorescent label) that binds to a therapeutic antibody (Molecular Biology of the Cell, Vol. 15, 5268-5282, December 2004), or (3) Mab-ZAP assay in which immunotoxin that binds to a therapeutic antibody is used to inhibit cell proliferation when incorporated into a cell (Bio Techniques 28: 162-165, 162-165, January 2000). As the immunotoxin, a recombinant complex protein of a catalytic region of diphtheria toxin and protein G can be used.

The antitumor activity of the anti-HER3 antibody can be confirmed *in vitro* by measuring cell proliferation inhibitory activity. For example, a cancer cell line overexpressing a target protein of the anti-HER3 antibody is cultured, the anti-HER3 antibody is added to the culture system in various concentrations, and inhibitory activities against focus formation, colony formation, and spheroid proliferation can be measured. *In vivo,* for example, the antibody is administered to a nude mouse into which a cancer cell line highly expressing a target protein has been transplanted, and the antitumor activity can be confirmed by measuring change of cancer cells.

Although it is preferable that the anti-HER3 antibody itself has the antitumor effect, since a compound exhibiting the antitumor effect is bound in the antibody-drug conjugate of the present invention, the antitumor effect of the anti-HER3 antibody itself is not essential. For the purpose of specifically/selectively exhibiting the cytotoxicity of an antitumor compound in a cancer cell, it is important, and preferable that the anti-HER3 antibody has a property of being internalized to translocate into the cancer cell.

The anti-HER3 antibody used in the present invention can be obtained by any known means. For example, the anti-HER3 antibody can be obtained by immunizing an animal with HER3 or an optional polypeptide selected from an amino acid sequence of HER3 used as an antigen by a method usually employed in this field, collecting an antibody thus produced in the living body, and purifying the resultant. The origin of the antigen is not limited to a human, but an antigen derived from an animal other than a human, such as a mouse or a rat, can be used for immunizing an animal. In this case, an anti-HER3 antibody applicable to a human disease can be selected by testing cross-reactivity between an antibody that binds to a heteroantigen thus obtained and a human antigen.

Alternatively, a monoclonal antibody can be obtained by establishing a hybridoma by fusing an antibody-producing cell for producing an antibody against an antigen, and a myeloma cell in accordance with a known method (such as Kohler and Milstein, Nature (1975) 256, p. 495-497; or Kennet, R. ed., Monoclonal Antibodies, p. 365-567, Plenum Press, N. Y. (1980)).

It is noted that an antigen can be obtained by causing a host cell to produce a gene encoding an antigen protein by genetic engineering. Specifically, a vector capable of expressing the antigen gene is produced, the resultant is introduced into a host cell to express the gene, and the thus expressed antigen is purified. The antibody can be obtained also by immunizing an animal with the antigen expressing cell or a cell line expressing the antigen, obtained by genetic engineering.

The anti-HER3 antibody used in the present invention is preferably a gene recombinant antibody artificially modified for the purpose of reducing heteroantigenicity to a human or the like, such as a chimeric antibody or a humanized antibody, or preferably an antibody having only a gene sequence of a human-derived antibody, namely, a human antibody.

Such an antibody can be produced by any known method.

An example of the chimeric antibody includes an antibody in which the variable region and the constant region of the antibody are heterogeneous to each other, such as a chimeric antibody obtained by binding a mouseor rat-derived antibody variable region to a human-derived constant region (Proc. Natl. Acad. Sci. U. S. A., 81, 6851-6855, (1984)).

Examples of the humanized antibody include an antibody in which only a complementarity determining region (CDR) of a heteroantibody is incorporated into a human-derived antibody (Nature (1986) 321, p. 522-525), an antibody in which a part of framework amino acid residues of a heteroantibody are transplanted into a human antibody in addition to the sequence of the CDR of the heteroantibody (International Publication No. WO90/07861) by the CDR transplantation method, and an antibody humanized by gene conversion mutagenesis strategy (U.S. Patent No. 5821337).

Examples of the human antibody also include antibodies produced by using human antibody-producing mice having human chromosome fragments containing genes of a heavy chain and a light chain of a human antibody (see Tomizuka, K. et al., Nature Genetics (1997) 16, p. 133-143; Kuroiwa, Y. et al., Nucl. Acids Res. (1998) 26, p. 3447-3448; Yoshida, H. et al., Animal Cell Technology: Basic and Applied Aspects vol.10, p.69-73 (Kitagawa, Y., Matsuda, T. and Iijima, S. eds.), Kluwer Academic Publishers, 1999; Tomizuka, K. et al., Proc. Natl. Acad. Sci. USA (2000) 97, p. 722-727, and the like). Alternatively, examples also include antibodies obtained by phage display selected from a human antibody library (see Wormstone, I. M. et al., Investigative Ophthalmology & Visual Science. (2002) 43 (7), p. 2301-2308; Carmen, S. et al., Briefings in Functional Genomics and Proteomics (2002), 1(2), p. 189-203; Siriwardena, D. et al., Ophthalmology (2002) 109(3), p. 427-431, and the like).

The anti-HER3 antibody used in the present invention encompasses a modified product of the antibody. The modified product means a product obtained by chemically or biologically modifying the antibody of the present invention. Examples of a chemically modified product include chemically modified products having a bond of a chemical moiety to an amino acid skeleton, and having a bond of a chemical moiety to a N-linked or O-linked carbohydrate chain. Examples of a biologically modified product include post-translationally modified products (such as those obtained by adding a N-linked or O-linked sugar chain, processing the N-terminus or C-terminus, deamidation, isomerizing aspartic acid, or oxidizing methionine), and products in which a methionine residue is added to the N-terminus by expression using a prokaryote host cell. Furthermore, such modified products encompass those labeled for enabling detection or isolation of the anti-HER3 antibody or antigen used in the present invention, such as an enzyme-labeled product, a fluorescence-labeled product, and an affinity-labeled product. Such modified products of the anti-HER3 antibody used in the present invention are useful for improvement of the stability and retentivity in blood of the antibody, reduction of antigenicity, and detection or isolation of the antibody or antigen.

Furthermore, the antibody-dependent cytotoxic activity can be increased by controlling (for example, glycosylating, or defucosylating) sugar chain modification attached to the anti-HER3 antibody used in the present invention. Known examples of the technique for controlling sugar chain modification of an antibody include, but are not limited to, International Publication No. WO99/54342, International Publication No. WO00/61739, and International Publication No. WO02/31140. The anti-HER3 antibody used in the present invention encompasses the antibody having controlled sugar chain modification.

It is known that an antibody produced in mammal cultured cells lacks a lysine residue at the carboxyl terminus of the heavy chain thereof (Journal of Chromatography A, 705: 129-134 (1995)), and it is known that two amino acid residues of glycine and lysine at the carboxyl terminus of the heavy chain are also lacked, and a proline residue at the carboxyl terminus is newly amidated (Analytical Biochemistry, 360: 75-83 (2007)). These lacks and modifications of the heavy chain sequence, however, do not affect the ability to bind its antigen and the effector function (such as activation of a complement, and antibody-dependent cytotoxic effect) of the antibody. Accordingly, the anti-HER3 antibody used in the present invention encompasses antibodies having these modifications, and functional fragments of the antibodies, and also encompasses a deletion product lacking in one or two amino acids in the carboxyl terminus of the heavy chain, the deletion product amidated (for example, having an amidated proline residue at the carboxyl terminus site in the heavy chain), and the like. The deletion product at the carboxyl terminus of the heavy chain of the anti-HER3 antibody used in the present invention is, however, not limited to the above-described types as long as the ability to bind its antigen and the effector function are retained. The two heavy chains contained in the anti-HER3 antibody used in the present invention may be any one of heavy chains selected from the group consisting of a full-length heavy chain, and the above-described deletion products, or may be a combination of any two of these. The quantity ratio of each of the deletion products can be affected by the type and culture conditions of mammal cultured cells producing the anti-HER3 antibody used in the present invention, and the anti-HER3 antibody used in the present invention preferably lacks one amino acid residue at the carboxyl terminus in both the two heavy chains.

In the present description, the term "functional fragment of an antibody", also called "antigen-binding fragment of an antibody", is used to mean a partial fragment of the antibody having binding activity against an antigen, and includes Fab, F(ab')2, scFv, a diabody, a linear antibody and a multispecific antibody formed from antibody fragments, and the like. Fab', which is a monovalent fragment of antibody variable regions obtained by treating F(ab')2 under reducing conditions, is also included in the antigen-binding fragment of an antibody. However, the antigen-binding fragment of an antibody is not

limited to these molecules, as long as the antigen-binding fragment has antigen-binding ability. These antigen-binding fragments include not only those obtained by treating a full-length molecule of an antibody protein with an appropriate enzyme, but proteins produced in appropriate host cells using a genetically engineered antibody gene.

Isotypes of the anti-HER3 antibody used in the present invention can be, for example, IgG (IgG1, IgG2, IgG3, or IgG4) or the like, and is preferably IgG1 or IgG2. Furthermore, variants of these can be also used as the anti-HER3 antibody of the present invention.

Examples of the anti-HER3 antibody usable in the present invention include patritumab (U3-1287), U1-59 (International Publication No. WO2007/077028), AV-203 (International Publication No. WO2011/136911), LJM-716 (International Publication No. WO2012/022814), duligotumab (MEHD-7945A) (International Publication No. WO2010/108127), istiratumab (MM-141) (International Publication No. WO2011/047180), lumretuzumab (RG-7116) (International Publication No. WO2014/108484), setibantumab (MM-121) (International Publication No. WO2008/100624), REGN-1400 (International Publication No. WO2013/048883), ZW-9 (International Publication No. WO2013/063702), and variants, active fragments, modified products and the like thereof, and the antibody is preferably patritumab, or U1-59. These anti-HER3 antibodies can be produced by methods described in the respective literatures.

3. Antibody-Drug Conjugate

As used herein, the term "antibody-drug conjugate" refers to a complex in which a drug having cytotoxicity is conjugated to an antibody via a linker. Examples of the antibody-drug conjugate include those described in U.S. Patent No. 6214345, International Publication No. WO2002/083067, International Publication No. WO2003/026577, International Publication No. WO2004/054622, International Publication No. WO2005/112919, International Publication No. WO2006/135371, International Publication No. WO2007/112193, International Publication No. WO2008/033891, International Publication No. WO2009/100194, International Publication No. WO2009/134976, International Publication No. WO2009/134977, International Publication No. WO2010/093395, International Publication No. WO2011/130613, International Publication No. WO2011/130616, International Publication No. WO2013/055993, International Publication No. WO2014/057687, International Publication No. WO2014/061277, International Publication No. WO2014/107024, International Publication No. WO2014/134457, and International Publication No. WO2014/145090, and the antibody-drug conjugate is preferably those described in International Publication No. WO2014/057687, and International Publication No. WO2014/061277, and more preferably that described in International Publication No. WO2014/057687. These antibody-drug conjugates can be produced by methods respectively described in the above-described literatures.

The drug having cytotoxicity is not especially limited as long as it has an antitumor effect, and has a substituent or a partial structure capable of binding to a linker, and examples include camptothecin, calichemicin, doxorubicin, daunorubicin, mitomycin C, bleomycin, cyclocytidine, vincristine, vinblastine, methotrexate, cisplatin, auristatin E, maytansine, paclitaxel, pyrrolobenzodiazepine, and derivatives thereof. The drug is preferably a campotecin derivative, and more preferably an exatecan derivative. Exatecan that is topoisomerase I inhibitor (IUPAC name: (1S,9S)-1-amino-9-ethyl-5-fluoro-1,2,3,9,12,15-hexahydro-9-hydroxy-4-methyl-10H,13H-benzo[de]pyrano[3',4':6,7]indolizino[1,2-b]quinoline-10,13-dione (which can be expressed by a chemical name: ((1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione)) is a compound represented by the following formula:

[Formula 17]

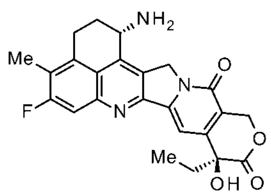

As used herein, the term "drug-linker" refers to a part corresponding to the drug and a linker portion in the antibody-drug conjugate, namely, a partial structure excluding the antibody in the antibody-drug conjugate.

As used herein, the term "anti-HER3 antibody-drug conjugate" refers to an antibody-drug conjugate using the anti-HER3 antibody as the antibody of an antibody-drug conjugate. Examples of the anti-HER3 antibody-drug conjugate include those described in International Publication No. WO2012/019024, International Publication No. WO2012/064733, and International Publication No. WO2015/155998, and the conjugate is preferably that described in International Publication No. WO2015/155998. These anti-HER3 antibody-drug conjugates can be produced by methods respectively described in the above-described literatures.

An anti-HER3 antibody-drug conjugate more preferably used in the present invention is an anti-HER3 antibody-drug conjugate in which a drug-linker represented by the following formula:

[Formula 18]

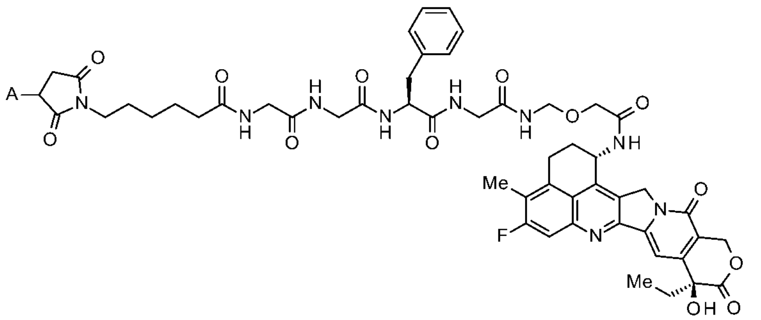

wherein A represents a connecting position to an anti-HER3 antibody or a functional fragment thereof, is conjugated to the anti-HER3 antibody or functional fragment thereof (preferably an anti-HER3 antibody) via a thioether bond. This drug-linker is conjugated to a thiol group (namely, a sulfur atom of a cysteine residue) generated in disulfide bond sites between the chains of the antibody (two sites between the heavy chains, and two sites between the heavy chain and the light chain).

The anti-HER3 antibody-drug conjugate more preferably used in the present invention can be represented also by the following formula:

[Formula 19]

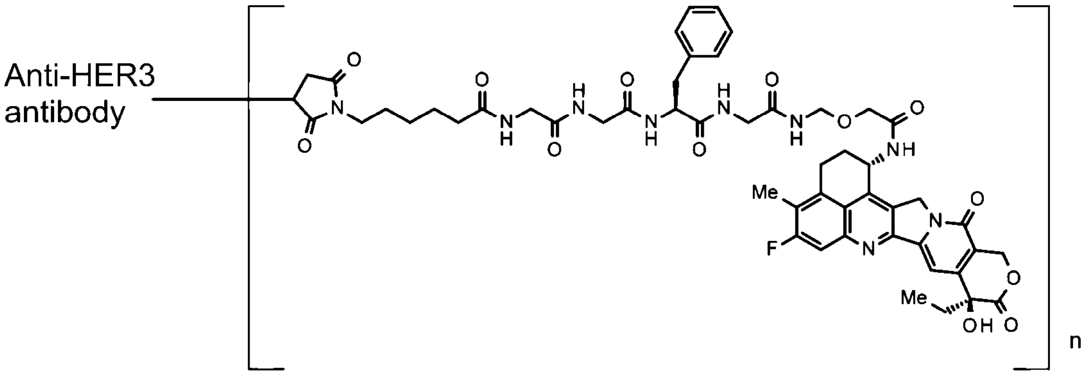

wherein 'anti-HER3 antibody' in the formula is an anti-HER3 antibody or a functional fragment thereof (preferably an anti-HER3 antibody); the drug-linker is conjugated to the antibody via a thioether bond.

Furthermore, the meaning of n is the same as that of what is called the average number of conjugated drug molecules (DAR; Drug-to-Antibody Ratio), and represents the average number of units of the drug-linker conjugated per antibody molecule. In the present invention, the "average number of conjugated drug molecules" is also called the drug-to-antibody ratio (DAR), and refers to the average number of drug molecules (or units of a drug-linker) that are conjugating per antibody molecule in a composition of antibody-drug conjugates.

An anti-HER3 antibody-drug conjugate more preferably used in the present invention translocates into a tumor cell, and then is cleaved at the linker portion to release a compound represented by the following formula:

[Formula 20]

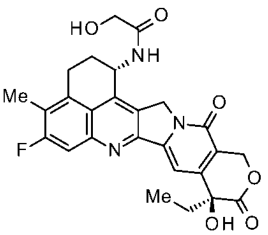

The compound seems to be a core part of the antitumor activity of the anti-HER3 antibody-drug conjugate more preferably used in the present invention, and has been confirmed to have topoisomerase I inhibitory effect (reference: Ogitani Y. et al., Clinical Cancer Research, 2016, Oct 15; 22 (20): 5097-5108, Epub 2016 Mar 29).

In some embodiments, the anti-HER3 antibody-drug conjugate used in the present invention is patritumab deruxtecan.

It is noted that the anti-HER3 antibody-drug conjugate used in the present invention has a bystander antitumor effect (reference: Ogitani Y. et al., Cancer Science (2016) 107, 1039-1046).

This bystander antitumor effect is exhibited when the anti-HER3 antibody-drug conjugate used in the present invention is internalized into a target expressing cancer cell, and then, the compound is liberated therefrom to exhibit the antitumor effect on nearby cancer cells not expressing the target.

This bystander antitumor effect is exhibited as an excellent antitumor effect also when the anti-HER3 antibody-drug conjugate of the present invention is used in combination with a RASG12C inhibitor.

The anti-HER3 antibody or functional fragment thereof used in the present invention is preferably an antibody or functional fragment thereof (preferably an anti-HER3 antibody) comprising: a heavy chain comprising CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and a light chain comprising CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2. More preferably, the anti-HER3 antibody or functional fragment thereof is an antibody or functional fragment thereof (preferably an antibody) comprising a heavy chain comprising a heavy chain variable region consisting of an amino acid sequence of amino acid residues 1 to 117 in SEQ ID NO: 1, and a light chain comprising a light chain variable region consisting of an amino acid sequence of amino acid residues 1 to 113 in SEQ ID NO: 2, and further preferably, is an antibody or functional fragment thereof (preferably an anti-HER3 antibody) comprising a heavy chain consisting of an amino acid sequence of SEQ ID NO: 1, and a light chain consisting of an amino acid sequence of SEQ ID NO: 2, or an antibody or functional fragment thereof (preferably an anti-HER3 antibody) that lacks a lysine residue at the carboxyl terminus of the heavy chain in the antibody.

A drug-linker intermediate used in the production of the anti-HER3 antibody-drug conjugate is represented by the following formula:

[Formula 21]

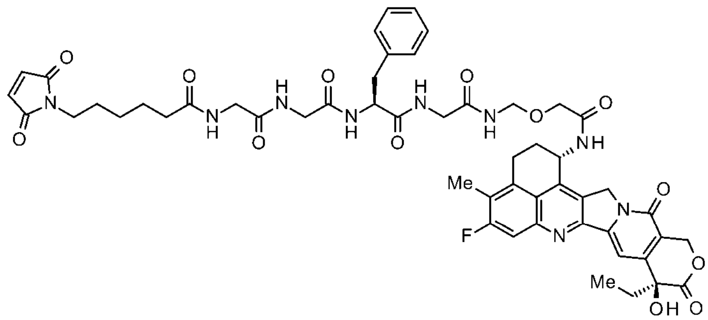

The drug-linker intermediate can be represented by a chemical name, N-[6-(2,5-dioxo-2,5-dihydro-1H-pyrrole-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycineamide, and can be produced referring to description given in International Publication No. WO2014/057687

and International Publication No. WO2015/155998.

The anti-HER3 antibody-drug conjugate preferably used in the present invention can be produced by reacting the above-described drug-linker intermediate with an anti-HER3 antibody having a thiol group (or also called a sulfhydryl group).

The anti-HER3 antibody having a sulfhydryl group can be obtained by any method known to those skilled in the art (Hermanson, G. T, Bioconjugate Techniques, pp. 56-136, pp. 456-493, Academic Press (1996)). For example, a reducing agent such as tris(2-carboxyethyl)phosphine hydrochloride (TCEP) is used in an amount of 0.3 to 3 mole equivalents per interchain disulfide within the antibody, and is reacted with the anti-HER3 antibody in a buffer containing a chelating agent such as ethylenediaminetetraacetic acid (EDTA), and thus, an anti-HER3 antibody having a sulfhydryl group in which the interchain disulfide within the antibody is partially or completely reduced can be obtained.

Alternatively, an anti-HER3 antibody-drug conjugate in which 2 to 8 drugs are conjugated per antibody can be produced by using a drug-linker intermediate in an amount of 2 to 20 mole equivalents per anti-HER3 antibody having a sulfhydryl group.

The average drug-to-antibody ratio per antibody molecule in the thus produced anti-HER3 antibody-drug conjugate can be obtained by, for example, a method in which it is calculated by measuring UV absorbances at the two wavelengths of 280 nm and 370 nm of the anti-HER3 antibody-drug conjugate and a conjugation precursor thereof (UV method), or a method in which it is calculated by quantitatively determining, by HPLC measurement, respective fragments obtained by treatment of the antibody-drug conjugate with a reducing agent (HPLC method).

The conjugation of the anti-HER3 antibody and the drug-linker intermediate, and the calculation of the average drug-to-antibody ratio per antibody molecule in the anti-HER3 antibody-drug conjugate can be performed referring to the description given in International Publication No. WO2015/155998, or the like.

The average number of units of the drug-linker conjugated per antibody molecule in the anti-HER3 antibody-drug conjugate used in the present invention is preferably 2 to 8, more preferably 3 to 8, further preferably 7 to 8, further preferably 7.5 to 8, and still further preferably about 8. In other embodiments, the number of the drug or the drug-linker conjugated per antibody molecule in the anti-HER3 antibody-drug conjugate is an integer in the range from preferably 2 to 8, more preferably selected from 2, 4, 6, or 8, and most preferably 8.

The anti-HER3 antibody-drug conjugate can be produced referring to the description given in International Publication No. WO2015/155998, or the like.

The anti-HER3 antibody-drug conjugate used in the present invention is known in the art. Examples of such anti-HER3 antibody-drug conjugates include, but are not limited to, DB-1310, IBI-133, SHR-A2009, AMT-562, and HMBD-501. Anti-HER3 antibody-drug conjugates are also described in US2021/0353764(WO2020063676), US2024/0026028 (WO2022078425), WO2023143365, WO2023041006, WO2023088382, WO2023125530, WO2023138635, WO2023143263, WO2023208216, WO2024077277, WO2024078449, WO2024083166, and WO2024088388, all of which are incorporated herein by reference.

4. Medicament

Now, a pharmaceutical product and a method of treatment characterized in that the anti-HER3 antibody-drug conjugate of the present invention and the RASG12C inhibitor are administered in combination will be described.

The pharmaceutical product and the method of treatment of the present invention may be characterized in that the anti-HER3 antibody-drug conjugate and the RASG12C inhibitor are comprised in different formulations as active ingredients, and are administered at the same time (a person skilled in the art would naturally understand that "at the same time" may be or may not be "at about the same time") or at different times, or may be characterized in that the anti-HER3 antibody-drug conjugate and the RASG12C inhibitor are comprised in a single formulation as active ingredients to be administered.

In the case where the anti-HER3 antibody-drug conjugate and the RASG12C inhibitor are comprised in different formulations as active ingredients and are administered at different times (it may be different days), the order of administration thereof is not particularly limited. Since different formulations are usually administered according to their respective administration methods, the frequency of administration thereof may be the same times or may be different times. Further, such separate preparations may be administered by the same administration method (administration route) or may be administered by different administration methods (administration routes). It is not necessary that the anti-HER3 antibody-drug conjugate and the RASG12C inhibitor exist in the body concurrently, and it is sufficient that the anti-HER3 antibody-drug conjugate and the RASG12C inhibitor are incorporated in the body within a certain period (e.g., for 1 month, preferably for 1 week, more preferably for several days, even more preferably for 1 day). Alternatively, when one of the active ingredients is administered, the other active ingredient may have already disappeared from the body.

The "pharmaceutical product" of the present invention is characterized in that the anti-HER3 antibody-drug conjugate and the RASG12C inhibitor are comprised in a single formulation as active ingredients to be administered.

The pharmaceutical product and the method of treatment of the present invention can be used for treating cancer,

can be used for treating preferably at least one cancer selected from the group consisting of breast cancer (including triple negative breast cancer, and luminal breast cancer), stomach cancer (also called gastric cancer), large intestine cancer (also called colorectal cancer, and including colon cancer and rectal cancer), lung cancer (including small cell lung cancer, and non-small-cell lung cancer), esophageal cancer, head and neck cancer (including salivary gland cancer, and pharyngeal cancer), gastroesophageal junction cancer, biliary tract cancer (including bile duct cancer), Paget disease, pancreatic cancer, ovarian cancer, uterine carcinosarcoma, urothelial cancer, prostate cancer, bladder cancer, gastrointestinal stromal tumor, cervical cancer, squamous cell carcinoma, peritoneal cancer, liver cancer, hepatocellular cancer, uterine cancer, kidney cancer, vulvar cancer, thyroid cancer, penile cancer, leukemia, malignant lymphoma, plasma cell neoplasm, myeloma, glioblastoma multiforme, sarcoma, osteosarcoma, and melanoma, and can be used for treating more preferably at least one cancer selected from the group consisting of breast cancer, stomach cancer, large intestine cancer, lung cancer, esophageal cancer, head and neck cancer, gastroesophageal junction cancer, biliary tract cancer, Paget disease, pancreatic cancer, ovarian cancer, bladder cancer, prostate cancer, uterine carcinosarcoma, gastrointestinal stromal tumor, kidney cancer, and sarcoma.

In another aspect, the pharmaceutical product and the method of treatment of the present invention can be used for treating at least one cancer selected from the group consisting of breast cancer, stomach cancer, large intestine cancer, lung cancer, esophageal cancer, head and neck cancer, gastroesophageal junction cancer, biliary tract cancer, Paget disease, pancreatic cancer, ovarian cancer, bladder cancer, prostate cancer, uterine carcinosarcoma, gastrointestinal stromal tumor, kidney cancer, and sarcoma.

In another aspect, the pharmaceutical product and the method of treatment of the present invention can be used for treating breast cancer.

In another aspect, the pharmaceutical product and the method of treatment of the present invention can be used for treating stomach cancer.

In another aspect, the pharmaceutical product and the method of treatment of the present invention can be used for treating lung cancer.

In another aspect, the pharmaceutical product and the method of treatment of the present invention can be used for treating non-small-cell lung cancer.

Cancers for which the pharmaceutical product and the method of treatment of the present invention can be used preferably express HER3, and more preferably highly express HER3.

The expression of HER3 can be confirmed by detection at the level of a gene product (protein) of HER3 by immunohistochemistry method (IHC), flow cytometer, Western blot method or the like, or by detection at the gene transcription level by *in situ* hybridization method (ISH) or quantitative PCR method (q-PCR). It can be determined by methods known to those skilled in the art whether or not HER3 is highly expressed.

Cancers for which the pharmaceutical product and the method of treatment of the present invention can be used preferably have KRAS, HRAS, and/or NRAS G12C mutation, and more preferably have KRAS G12C mutation.

It can be determined whether or not cancer contains KRAS, HRAS, or NRAS G12C mutation by evaluating a nucleotide sequence encoding KRAS, HRAS, or NRAS protein, evaluating an amino acid sequence of KRAS, HRAS, or NRAS protein, or evaluating properties of presumed KRAS, HRAS, or NRAS mutant protein. Sequences of wild-type human KRAS, HRAS, and NRAS are known in this technical field (for example, accession No. NP203524).

Methods for detecting mutation in the KRAS, HRAS, or NRAS nucleotide sequence are known to those skilled in the art. Examples of these methods include, but are not limited to, polymerase chain reaction - restriction enzyme fragment length polymorphism (PCR-RFLP) assay, polymerase chain reaction - single strand conformation polymorphism (PCR-SSCP) assay, real time PCR assay, PCR sequencing, mutant allele-specific PCR amplification (MASA) assay, direct sequencing, primer extension reaction, electrophoresis, oligonucleotide ligation assay, hybridization assay, TaqMan assay, SNP genotyping assay, high resolution melting assay, and microarray analysis. In some embodiments, a sample is evaluated for the KRAS, HRAS, or NRAS G12C mutation by real time PCR. In the real time PCR, a fluorescent probe specific to the KRAS, HRAS, or NRAS G12C mutation is used. When there is mutation, the probe binds thereto, and the fluorescence is detected. In some embodiments, the KRAS, HRAS, or NRAS G12C mutation is identified by direct sequencing method for a specific region (such as exon 2 and/or exon 3) of KRAS, HRAS, or NRAS gene. In this method, all possible mutations in the sequenced region are identified.

Methods for detecting mutation of KRAS, HRAS, or NRAS protein are known to those skilled in the art. An example of these methods includes, but is not limited to, detection of KRAS, HRAS, or NRAS mutant by using a binding agent specific to the mutant protein (such as an antibody), electrophoresis or Western blot of the protein, or direct sequencing of the peptide.

In a method for determining whether or not tumor or cancer contains KRAS, HRAS, or NRAS G12C mutation, various samples can be used. In some embodiments, the sample is collected from a subject having the tumor or cancer. In some embodiments, the sample is a fresh tumor/cancer sample. In some embodiments, the sample is a frozen tumor/cancer sample. In some embodiments, the sample is a formalin-fixed and paraffin-embedded sample. In some embodiments, the sample is a circulating tumor cell (CTC) sample. In some embodiments, the sample is prepared into a

cell lysate. In some embodiments, the sample is prepared into a DNA or RNA.

The term "subject" as used herein refers to a human or another animal in need of the treatment, and in one aspect, is a human in need of the treatment.

The antitumor effect of the pharmaceutical product and the method of treatment of the present invention can be confirmed by, for example, creating a model by transplanting a cancer cell into a test animal, and measuring reduction of the tumor volume and life extension effect obtained by using the pharmaceutical product and the method of treatment of the present invention. Then, when this antitumor effect is compared with the antitumor effect obtained by single administration with each of the antibody-drug conjugate and the RASG12C inhibitor used in the present invention, the combined effect of the anti-HER3 antibody-drug conjugate and the RASG12C inhibitor used in the present invention can be confirmed.

Furthermore, the antitumor effect of the pharmaceutical product and the method of treatment of the present invention can be confirmed, in a clinical test, by Response Evaluation Criteria in Solid Tumors (RECIST) method, WHO evaluation method, Macdonald evaluation method, weight measurement, and other methods, and complete response (CR), partial response (PR), progressive disease (PD), objective response rate (ORR), duration of response (DoR), progression-free survival (PFS), overall survival (OS), and the like can be used as indexes for the determination.

By any of the above-described methods, superiority of the antitumor effect of the pharmaceutical product and the method of treatment of the present invention to existing pharmaceutical products and methods of treatment for cancer can be confirmed.

The pharmaceutical product and the method of treatment of the present invention can delay the growth of cancer cells, can inhibit the proliferation thereof, and further can destroy cancer cells. Owing to these effects, a cancer patient can be freed from the symptoms of the cancer, and improve the QOL, and thus, the treatment effects can be achieved with the life of the cancer patient maintained. Even when the cancer cells are not destroyed, the cancer patient can achieve higher QOL and survive a longer period by inhibition and control of the cancer cell proliferation.

The pharmaceutical product of the present invention can not only be applied to a patient as systemic treatment but also be locally applied to a cancer tissue to expect the treatment effect.

The pharmaceutical product of the present invention can be administered with one or more pharmaceutically compatible ingredients contained therein. The pharmaceutically compatible ingredients can be appropriately selected, to be applied, from formulation additives and the like usually used in this field in accordance with doses and dosage concentrations of the anti-HER3 antibody-drug conjugate and the RASG12C inhibitor used in the present invention. For example, the anti-HER3 antibody-drug conjugate used in the present invention can be administered as a pharmaceutical product containing a buffer such as histidine buffer, an excipient such as sucrose or trehalose, and a surfactant such as polysorbate 80 or 20. The pharmaceutical product comprising the antibody-drug conjugate used in the present invention can be used preferably as an injection, more preferably as an aqueous injection or freeze-dried injection, and further preferably as a freeze-dried injection.

When the pharmaceutical product comprising the anti-HER3 antibody-drug conjugate used in the present invention is an aqueous injection, it can be preferably diluted with a proper diluent, and then administered by intravenous drip. Examples of the diluent include a glucose solution, and a saline solution, a preferable example includes a glucose solution, and a more preferable example includes a 5% glucose solution.

When the pharmaceutical product comprising the anti-HER3 antibody-drug conjugate used in the present invention is a freeze-dried injection, it can be preferably administered by intravenous drip after dissolving it in water for injection, and diluting a necessary amount thereof with a proper diluent. Examples of the diluent include a glucose solution, and a saline solution, a preferable example includes a glucose solution, and a more preferable example includes a 5% glucose solution.

The pharmaceutical product and the method of treatment of the present invention may further comprise a cancer treatment drug in addition to the anti-HER3 antibody-drug conjugate and the RASG12C inhibitor of the present invention. The pharmaceutical product and the method of treatment of the present invention can be administered concurrently with another cancer treatment drug, and thus, the antitumor effect can be enhanced. Another cancer treatment drug used for this purpose may be administered to the subject simultaneously with, separately from, or consecutively with the pharmaceutical product of the present invention, or may be administered at different administration intervals. Such a cancer treatment drug is not limited as long as it is a drug having antitumor activity, and for example, can be at least one selected from the group consisting of irinotecan (CPT-11), cisplatin, carboplatin, oxaliplatin, fluorouracil (5-FU), gemcitabine, capecitabine, paclitaxel, docetaxel, doxorubicin, epirubicin, cyclophosphamide, mitomycin C, a compounding agent of tegafur/gimeracil/oteracil, cetuximab, panitumumab, bevacizumab, ramucirumab, regorafenib, a compounding agent of trifluridine/tipiracil, gefitinib, erlotinib, afatinib, methotrexate, pemetrexed, tamoxifen, toremifene, fulvestrant, leuprorelin, goserelin, letrozole, anastrozole, progesterone formulation, trastuzumab, pertuzumab, and lapatinib.

The pharmaceutical product and the method of treatment of the present invention can be used in combination with radiation therapy. For example, a cancer patient receives radiation therapy before and/or after, or simultaneously with the treatment with the pharmaceutical product of the present invention.

The pharmaceutical product and the method of treatment of the present invention can be used as auxiliary

chemotherapy in combination with surgical operation. The pharmaceutical product of the present invention may be administered for the purpose of reducing the size of tumor before surgical operation (a treatment called neoadjuvant chemotherapy), or may be administered after surgical operation for the purpose of preventing tumor recurrence (a treatment called adjuvant chemotherapy). The pharmaceutical product and the method of treatment of the present invention can be used also as maintenance therapy.

In addition to these therapeutic uses, the treatment drug and the method of treatment of the present invention can be expected to exhibit preventive effects that, e.g., inhibit proliferation of, and even destroy, microscopic metastatic cancer cells. For example, inhibitory and destructive effects on cancer cells present in a body fluid in the process of metastasis, and inhibitory and destructive effects on microscopic cancer cells immediately after implantation in any tissue can be expected. Accordingly, inhibitory or preventive effects on cancer metastasis particularly after surgically removing the cancer can be expected.

The pharmaceutical product and the method of treatment of the present invention can not only be applied as systemic treatment for a patient but also be locally applied to a cancer tissue to expect the treatment effect.

The pharmaceutical product and the method of treatment of the present invention can be used preferably in a mammal, and can be used more preferably in a human.

The pharmaceutical product of the present invention can be administered as a pharmaceutical product comprising one or more pharmaceutically compatible ingredients. Doses and dosage concentrations of substances used in the pharmaceutical product of the present invention can be appropriately selected, to be applied, from formulation additives and the like usually used in this field. For example, the pharmaceutical product typically comprises one or more pharmaceutical carriers (such as a sterilized liquid). Here, a liquid encompasses, for example, water and oils (petroleum, and an animal-derived, plant-derived, and synthesis-derived oils). The oil may be, for example, peanut oil, soybean oil, mineral oil, sesame oil, or the like. Water is a more typical carrier in intravenous administration of the pharmaceutical product. A saline aqueous solution, a dextrose aqueous solution and a glycerol aqueous solution can be also used as the liquid carrier, particularly for a solution for injection. A proper pharmaceutical excipient can be appropriately selected from those known in this field. The pharmaceutical product can also comprise, if desired, a small amount of a moistening agent or an emulsifier, or a pH buffering agent. Examples of the proper pharmaceutical carrier are described in "Remington's Pharmaceutical Sciences" written by E. W. Martin. The formulation depends on the form of administration.

Various delivery systems are known, and can be used for administering the pharmaceutical product of the present invention. Examples of the introduction route include, but are not limited to, intradermal, intramuscular, intraperitoneal, intravenous, and subcutaneous routes. The administration can be performed by, for example, infusion or bolus injection. In a specific preferred embodiment, the antibody-drug conjugate is administered by infusion. The parenteral administration is a preferable administration route.

In a typical embodiment, the pharmaceutical product is prescribed in accordance with usual procedures as a pharmaceutical product compatible with intravenous administration to a human. Typically, a pharmaceutical product for intravenous administration is a solution in a sterilized isotonic aqueous buffer. If necessary, the pharmaceutical product can also comprise a solubilizing agent, and a local anesthetic (such as lignocaine) for relieving pain at the injection site. In general, the above-described ingredients are supplied in the form of a freeze-dried powder or an anhydrous concentrate in an airtightly sealed container, such as an ampoule or a sachet indicating the amount of an activator either separately, or in a mixed state in a unit dosage form. In an aspect in which the pharmaceutical product is administered by infusion, it can be administered with, for example, an injection bottle holding sterilized pharmaceutical grade water or saline. When the pharmaceutical is administered by injection, an ampoule of sterile water for injection or saline can be provided in such a manner that the aforementioned ingredients can be mixed before the administration.

The dose of the anti-HER3 antibody-drug conjugate used in the present invention is about 0.001 to 100 mg/kg per administration, and is preferably 1.6 mg/kg, 3.2 mg/kg, 4.8 mg/kg, 5.6 mg/kg, 6.4 mg/kg, 8.0 mg/kg, 9.6 mg/kg, or 12.8 mg/kg per administration.

The administration interval of the anti-HER3 antibody-drug conjugate used in the present invention is once per 1 to 180 days, and is preferably once a week (q1w), once every two weeks (q2w), once every three weeks (q3w), or once every four weeks (q4w), and is more preferably once every three weeks (q3w).

The RASG12C inhibitor used in the present invention can be administered at a dose of 0.1 mg to 3000 mg per administration, and can be preferably administered at a dose of 0.25 mg to 1000 mg per administration.

The RASG12C inhibitor of the present invention can be administered to a human at intervals of once or twice per 1 to 7 days, and can be administered preferably at intervals of once a day or twice a day.

When the RASG12C inhibitor used in the present invention is adagrasib or a pharmaceutically acceptable salt thereof, examples of the administration method include, but are not limited to, the following dosages and administrations.

For example, adagrasib can be orally administered at a dose of 600 mg per administration at intervals of twice daily.

As another dosage and administration, adagrasib can be orally administered at a dose of 400 mg per administration at intervals of twice daily.

As another dosage and administration, adagrasib can be orally administered at a dose of 600 mg per administration at intervals of once daily.

When the RASG12C inhibitor used in the present invention is sotorasib or a pharmaceutically acceptable salt thereof, examples of the administration method include, but are not limited to, the following dosages and administrations.

For example, sotorasib can be orally administered at a dose of 960 mg per administration at intervals of once daily.

As another dosage and administration, sotorasib can be orally administered at a dose of 480 mg per administration at intervals of once daily.

As another dosage and administration, sotorasib can be orally administered at a dose of 240 mg per administration at intervals of once daily.

As used herein, unless specifically stated otherwise and not limited in particular, and as long as it does not contradict technically and in the context, a term written in the singular form is considered to include the plural form, and vice versa.

[Examples]

The present invention will now be specifically described by way of examples below, and it is noted that the present invention is not limited to these. Furthermore, these should not be interpreted as limitation in any means.

Example 1: Preparation of Anti-HER3 Antibody-Drug Conjugate

In accordance with a production method described in International Publication No. WO2015/155998, an anti-HER3 antibody comprising a heavy chain consisting of an amino acid sequence represented by SEQ ID NO: 1 (Figure 1), and a light chain consisting of an amino acid sequence represented by SEQ ID NO: 2 (Figure 2) (referred to as the "anti-HER3 antibody (1)" in the present invention) was used to produce an anti-HER3 antibody-drug conjugate in which a drug-linker represented by the following formula:

[Formula 22]

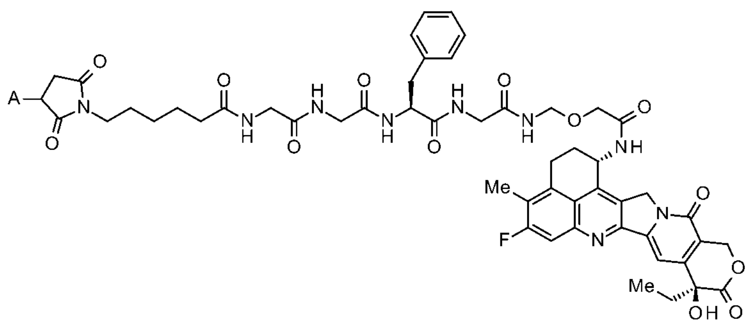

wherein A represents a connecting position to an antibody,
is conjugated to the anti-HER3 antibody via a thioether bond (referred to as the "HER3-ADC (1)" in the present invention). The average drug-to-antibody ratio per antibody molecule in the HER3-ADC (1) was 7.6.

Example 2: Antitumor Test (1)

Mouse: Female BALB/c nude mice of 5 to 6 week-old (Charles River Laboratories Japan, Inc.) were used in an experiment.

Measurement and calculation formula: In all studies, the long diameter and the short diameter of a tumor were measured with an electronic digital caliper (CD-15CX, Mitutoyo Corporation) twice a week to calculate the tumor volume ($mm^3$). The calculation formula was as follows:

$$\text{Tumor volume } (mm^3) = 1/2 \times \text{long diameter (mm)} \times [\text{short diameter (mm)}]^2$$

HER3-ADC (1) was diluted with ABS buffer (10 mM acetate buffer (pH 5.5), 5% sorbitol), and the resultant liquid was administered in an amount of 10 mL/kg to the tail vein.

Sotorasib was dissolved in 2% hydroxypropyl methylcellulose and 1% Tween 80, and the resultant liquid was orally administered in an amount of 10 mL/kg. Adagrasib was dissolved in a solution of 10% captisol, and 50 mM citrate buffer, pH 5.0, and the resultant liquid was orally administered in an amount of 10 mL/kg. NCI-H358 cells, that is, human lung cancer line purchased from ATCC (American Type Culture Collection) were suspended in 50% Matrigel matrix (Invitrogen), and 3 x 106 cells thus obtained were subcutaneously transplanted into a right side portion of the body of each of the female nude mice, and the resultant mice were randomly grouped 13 days after the transplantation (Day 0). HER3-ADC (1) was administered to the tail vein at a dose of 3 mg/kg on Day 0, Day 7, and Day 14. The RASG12C inhibitors were administered at a dose of 10 mg/kg for 3 weeks once a day and five times a week. Groups each given one of these agents, a group given both, and a solvent administration group as a control group were established.

Figure 3 illustrates the results of combined use of HER3-ADC (1) and sotorasib. The tumor growth inhibition (TGI) obtained on the final day of the test of single administration of sotorasib was 39%. The TGI obtained by single administration of HER3-ADC (1) was 68%. On the other hand, when HER3-ADC (1) and sotorasib were administered in combination, a tumor growth inhibitory effect significantly superior to that obtained by the single administration of sotorasib was obtained ($P < 0.001$ (calculated by Dunnett's test; the same applies below)). Furthermore, the tumor growth inhibition was higher (TGI of 85%) than that obtained by the single administration of HER3-ADC (1), and thus, the combined effect was strong. In the drawing, the abscissa indicates the number of days after the cell transplantation, and the ordinate indicates the tumor volume. Furthermore, a mouse belonging to the sotorasib administration group was found to have anal prolapse on Day 35, and hence was euthanized. Apart from this, no notable findings such as weight reduction were found in the group given the other agent or in the group given both agents. In the following evaluation examples related to the antitumor test, the test was performed by the method employed in this evaluation example unless otherwise stated.

Figure 4 illustrates the results of combined use of HER3-ADC (1) and adagrasib. The TGI obtained by single administration of adagrasib was 26%. The TGI obtained by single administration of HER3-ADC (1) was 68%. On the other hand, when HER3-ADC (1) and adagrasib were administered in combination, a tumor growth inhibitory effect significantly superior to that obtained by the single administration of adagrasib was obtained ($P < 0.001$). Besides, the tumor growth inhibition was higher (TGI of 81%) than that obtained by the single administration of HER3-ADC (1), and thus, the combined effect was strong. No notable findings such as weight reduction were found in any of the groups each given one of the agents or both.

[Sequence Listing Free Text]

SEQ ID NO: 1: amino acid sequence of heavy chain of anti-HER3 antibody
SEQ ID NO: 2: amino acid sequence of light chain of anti-HER3 antibody

## Claims

1. A pharmaceutical product comprising an anti-HER3 antibody-drug conjugate and a RASG12C inhibitor.

2. The pharmaceutical product according to claim 1, wherein the anti-HER3 antibody-drug conjugate is an anti-HER3 antibody-drug conjugate in which a drug-linker represented by the following formula:

[Formula 1]

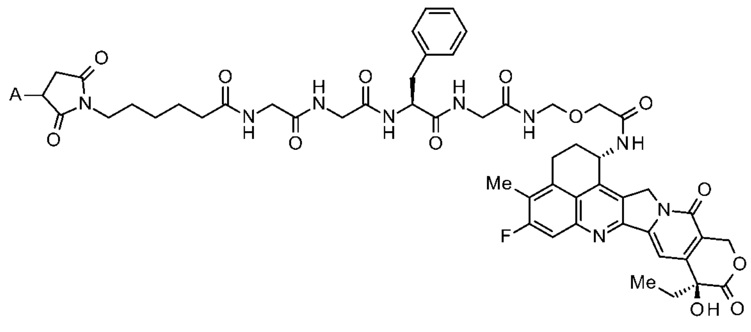

wherein A represents a connecting position to an anti-HER3 antibody or a functional fragment thereof,

is conjugated to the anti-HER3 antibody or functional fragment thereof via a thioether bond.

3. The pharmaceutical product according to claim 1 or 2, wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.

4. The pharmaceutical product according to claim 1 or 2, wherein the anti-HER3 antibody or functional fragment thereof comprises a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.

5. The pharmaceutical product according to claim 1 or 2, wherein the anti-HER3 antibody or functional fragment thereof comprises a heavy chain consisting of an amino acid sequence represented by SEQ ID NO: 1, and a light chain consisting of an amino acid sequence represented by SEQ ID NO: 2.

6. The pharmaceutical product according to claim 5, wherein the anti-HER3 antibody lacks a lysine residue at the carboxyl terminus of the heavy chain.

7. The pharmaceutical product according to any one of claims 1 to 6, wherein the average number of units of the drug-linker conjugated per antibody molecule in the anti-HER3 antibody-drug conjugate is in the range of 2 to 8.

8. The pharmaceutical product according to any one of claims 1 to 6, wherein the average number of units of the drug-linker conjugated per antibody molecule in the anti-HER3 antibody-drug conjugate is in the range of 3 to 8.

9. The pharmaceutical product according to any one of claims 1 to 6, wherein the average number of units of the drug-linker conjugated per antibody molecule in the anti-HER3 antibody-drug conjugate is in the range of 7 to 8.

10. The pharmaceutical product according to any one of claims 1 to 6, wherein the average number of units of the drug-linker conjugated per antibody molecule in the anti-HER3 antibody-drug conjugate is in the range of 7.5 to 8.

11. The pharmaceutical product according to any one of claims 1 to 6, wherein the average number of units of the drug-linker conjugated per antibody molecule in the anti-HER3 antibody-drug conjugate is 8.

12. The pharmaceutical product according to any one of claims 1 to 11, wherein the anti-HER3 antibody-drug conjugate is patritumab deruxtecan.

13. The pharmaceutical product according to any one of claims 1 to 12, wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

14. The pharmaceutical product according to any one of claims 1 to 12, wherein the RASG12C inhibitor is sotorasib (AMG510), adagrasib (MRTX849), or a pharmaceutically acceptable salt thereof.

15. The pharmaceutical product according to any one of claims 1 to 12, wherein the RASG12C inhibitor is sotorasib (AMG510), or a pharmaceutically acceptable salt thereof.

16. The pharmaceutical product according to any one of claims 1 to 12, wherein the RASG12C inhibitor is adagrasib (MRTX849), or a pharmaceutically acceptable salt thereof.

17. The pharmaceutical product according to any one of claims 1 to 16, wherein the antibody-drug conjugate and the RASG12C inhibitor are comprised in different formulations, and are to be administered at the same time or at different times.

18. The pharmaceutical product according to any one of claims 1 to 17, for use in treating cancer.

**19.** The pharmaceutical product according to any one of claims 1 to 18, for use in treating at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.

**20.** The pharmaceutical product according to claim 19, for use in treating breast cancer.

**21.** The pharmaceutical product according to claim 19, for use in treating stomach cancer.

**22.** The pharmaceutical product according to claim 19, for use in treating lung cancer.

**23.** The pharmaceutical product according to claim 22, wherein the lung cancer is non-small-cell lung cancer.

**24.** The pharmaceutical product according to any one of claims 1 to 23, wherein the pharmaceutical product is a pharmaceutical composition.

**25.** An anti-HER3 antibody-drug conjugate, for use in combination with a RASG12C inhibitor, in treating cancer.

**26.** The antibody-drug conjugate according to claim 25, wherein the anti-HER3 antibody-drug conjugate is an anti-HER3 antibody-drug conjugate in which a drug-linker represented by the following formula:

[Formula 2]

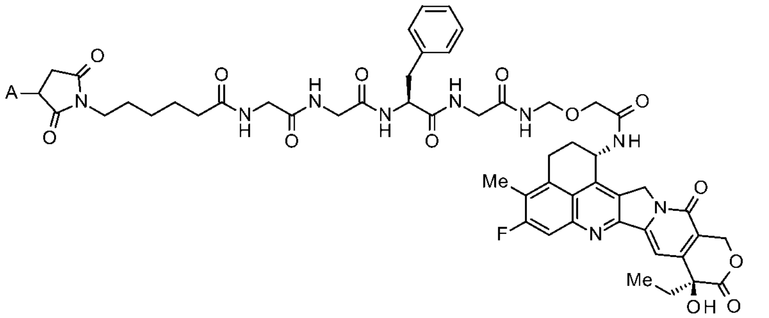

wherein A represents a connecting position to an anti-HER3 antibody or a functional fragment thereof, is conjugated to the anti-HER3 antibody or functional fragment thereof via a thioether bond.

**27.** The antibody-drug conjugate according to claim 25 or 26, wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.

**28.** The antibody-drug conjugate according to claim 25 or 26, wherein the anti-HER3 antibody or functional fragment thereof comprises a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.

**29.** The antibody-drug conjugate according to claim 25 or 26, wherein the anti-HER3 antibody or functional fragment thereof comprises a heavy chain consisting of an amino acid sequence represented by SEQ ID NO: 1, and a light chain consisting of an amino acid sequence represented by SEQ ID NO: 2.

**30.** The antibody-drug conjugate according to claim 29, wherein the anti-HER3 antibody lacks a lysine residue at the carboxyl terminus of the heavy chain.

**31.** The antibody-drug conjugate according to any one of claims 25 to 30, wherein the average number of units of the drug-linker conjugated per antibody molecule in the anti-HER3 antibody-drug conjugate is in the range of 2 to 8.

**32.** The antibody-drug conjugate according to any one of claims 25 to 30, wherein the average number of units of the drug-linker conjugated per antibody molecule in the anti-HER3 antibody-drug conjugate is in the range of 3 to 8.

**33.** The antibody-drug conjugate according to any one of claims 25 to 30, wherein the average number of units of the drug-linker conjugated per antibody molecule in the anti-HER3 antibody-drug conjugate is in the range of 7 to 8.

**34.** The antibody-drug conjugate according to any one of claims 25 to 30, wherein the average number of units of the drug-linker conjugated per antibody molecule in the anti-HER3 antibody-drug conjugate is in the range of 7.5 to 8.

**35.** The antibody-drug conjugate according to any one of claims 25 to 30, wherein the average number of units of the drug-linker conjugated per antibody molecule in the anti-HER3 antibody-drug conjugate is 8.

**36.** The antibody-drug conjugate according to any one of claims 25 to 35, wherein the anti-HER3 antibody-drug conjugate is patritumab deruxtecan.

**37.** The antibody-drug conjugate according to any one of claims 25 to 36, wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

**38.** The antibody-drug conjugate according to any one of claims 25 to 36, wherein the RASG12C inhibitor is sotorasib (AMG510), adagrasib (MRTX849), or a pharmaceutically acceptable salt thereof.

**39.** The antibody-drug conjugate according to any one of claims 25 to 36, wherein the RASG12C inhibitor is sotorasib (AMG510), or a pharmaceutically acceptable salt thereof.

**40.** The antibody-drug conjugate according to any one of claims 25 to 36, wherein the RASG12C inhibitor is adagrasib (MRTX849), or a pharmaceutically acceptable salt thereof.

**41.** The antibody-drug conjugate according to any one of claims 25 to 40, wherein the antibody-drug conjugate and the RASG12C inhibitor are comprised in different formulations, and are administered at the same time or at different times.

**42.** The antibody-drug conjugate according to any one of claims 25 to 41, wherein the cancer is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.

**43.** The antibody-drug conjugate according to claim 42, wherein the cancer is breast cancer.

**44.** The antibody-drug conjugate according to claim 42, wherein the cancer is stomach cancer.

**45.** The antibody-drug conjugate according to claim 42, wherein the cancer is lung cancer.

**46.** The antibody-drug conjugate according to claim 45, wherein the lung cancer is non-small-cell lung cancer.

**47.** A method of treatment for a disease, comprising administering an anti-HER3 antibody-drug conjugate and a RASG12C inhibitor in combination to a subject in need of treatment.

**48.** The method of treatment according to claim 47,

wherein the anti-HER3 antibody-drug conjugate is an anti-HER3 antibody-drug conjugate in which a drug-linker represented by the following formula:

[Formula 3]

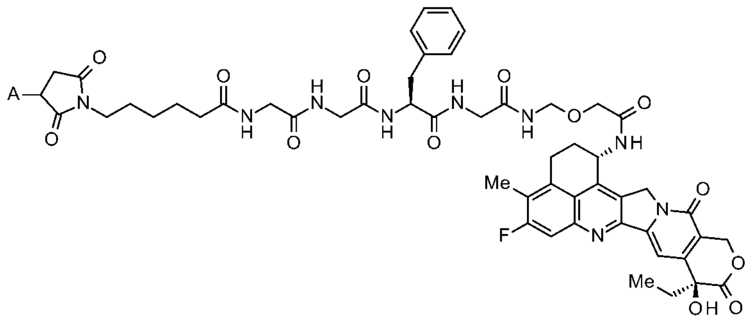

wherein A represents a connecting position to an anti-HER3 antibody or a functional fragment thereof, is conjugated to the anti-HER3 antibody or functional fragment thereof via a thioether bond.

**49.** The method of treatment according to claim 47 or 48, wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.

**50.** The method of treatment according to claim 47 or 48, wherein the anti-HER3 antibody or functional fragment thereof is an antibody comprising a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.

**51.** The method of treatment according to claim 47 or 48, wherein the anti-HER3 antibody or functional fragment thereof comprises a heavy chain consisting of an amino acid sequence represented by SEQ ID NO: 1, and a light chain consisting of an amino acid sequence represented by SEQ ID NO: 2.

**52.** The method of treatment according to claim 51, wherein the anti-HER3 antibody lacks a lysine residue at the carboxyl terminus of the heavy chain.

**53.** The method of treatment according to any one of claims 47 to 52, wherein the average number of units of the drug-linker conjugated per antibody molecule in the anti-HER3 antibody-drug conjugate is in the range of 2 to 8.

**54.** The method of treatment according to any one of claims 47 to 52, wherein the average number of units of the drug-linker conjugated per antibody molecule in the anti-HER3 antibody-drug conjugate is in the range of 3 to 8.

**55.** The method of treatment according to any one of claims 47 to 52, wherein the average number of units of the drug-linker conjugated per antibody molecule in the anti-HER3 antibody-drug conjugate is in the range of 7 to 8.

**56.** The method of treatment according to any one of claims 47 to 52, wherein the average number of units of the drug-linker conjugated per antibody molecule in the anti-HER3 antibody-drug conjugate is in the range of 7.5 to 8.

**57.** The method of treatment according to any one of claims 47 to 52, wherein the average number of units of the drug-linker conjugated per antibody molecule in the anti-HER3 antibody-drug conjugate is 8.

**58.** The method of treatment according to any one of claims 47 to 57, wherein the anti-HER3 antibody-drug conjugate is patritumab deruxtecan.

**59.** The method of treatment according to any one of claims 47 to 58, wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

**60.** The method of treatment according to any one of claims 47 to 58, wherein the RASG12C inhibitor is sotorasib (AMG510), adagrasib (MRTX849), or a pharmaceutically acceptable salt thereof.

**61.** The method of treatment according to any one of claims 47 to 58, wherein the RASG12C inhibitor is sotorasib (AMG510), or a pharmaceutically acceptable salt thereof.

**62.** The method of treatment according to any one of claims 47 to 58, wherein the RASG12C inhibitor is adagrasib (MRTX849), or a pharmaceutically acceptable salt thereof.

**63.** The method of treatment according to any one of claims 47 to 62, wherein the antibody-drug conjugate and the RASG12C inhibitor are comprised in different formulations, and are administered at the same time or at different times.

**64.** The method of treatment according to any one of claims 47 to 63, wherein the disease is cancer.

**65.** The method of treatment according to any one of claims 47 to 64, wherein the disease is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.

**66.** The method of treatment according to claim 65, wherein the disease is breast cancer.

**67.** The method of treatment according to claim 65, wherein the disease is stomach cancer.

**68.** The method of treatment according to claim 65, wherein the disease is lung cancer.

**69.** The method of treatment according to claim 68, wherein the lung cancer is non-small-cell lung cancer.

**70.** The pharmaceutical product according to claim 1, wherein the anti-HER3 antibody-drug conjugate is represented by the following formula:

[Formula 4]

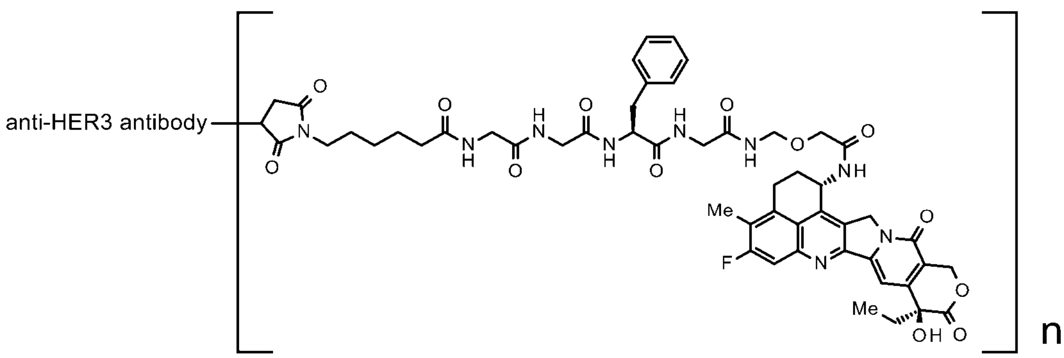

wherein 'anti-HER3 antibody' in the formula is an anti-HER3 antibody or a functional fragment thereof; a drug linker is conjugated to the antibody or functional fragment thereof via a thioether bond; and n represents drug-to-antibody ratio.

**71.** The pharmaceutical product according to claim 70, wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.

**72.** The pharmaceutical product according to claim 70, wherein the anti-HER3 antibody or functional fragment thereof comprises a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.

**73.** The pharmaceutical product according to claim 70, wherein the anti-HER3 antibody or functional fragment thereof comprises a heavy chain consisting of an amino acid sequence represented by SEQ ID NO: 1, and a light chain consisting of an amino acid sequence represented by SEQ ID NO: 2.

**74.** The pharmaceutical product according to claim 73, wherein the anti-HER3 antibody lacks a lysine residue at the carboxyl terminus of the heavy chain.

**75.** The pharmaceutical product according to any one of claims 70 to 74, wherein n is 2 to 8.

**76.** The pharmaceutical product according to any one of claims 70 to 74, wherein n is 3 to 8.

**77.** The pharmaceutical product according to any one of claims 70 to 74, wherein n is 7 to 8.

**78.** The pharmaceutical product according to any one of claims 70 to 74, wherein n is 7.5 to 8.

**79.** The pharmaceutical product according to any one of claims 70 to 74, wherein n is 8.

**80.** The pharmaceutical product according to any one of claims 70 to 79, wherein the anti-HER3 antibody-drug conjugate is patritumab deruxtecan.

**81.** The pharmaceutical product according to any one of claims 70 to 80, wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

**82.** The pharmaceutical product according to any one of claims 70 to 80, wherein the RASG12C inhibitor is sotorasib (AMG510), adagrasib (MRTX849), or a pharmaceutically acceptable salt thereof.

**83.** The pharmaceutical product according to any one of claims 70 to 80, wherein the RASG12C inhibitor is sotorasib (AMG510), or a pharmaceutically acceptable salt thereof.

**84.** The pharmaceutical product according to any one of claims 70 to 80, wherein the RASG12C inhibitor is adagrasib (MRTX849), or a pharmaceutically acceptable salt thereof.

**85.** The pharmaceutical product according to any one of claims 70 to 84, wherein the antibody-drug conjugate and the RASG12C inhibitor are comprised in different formulations, and are to be administered at the same time or at different times.

**86.** The pharmaceutical product according to any one of claims 70 to 85, for use in treating cancer.

**87.** The pharmaceutical product according to any one of claims 70 to 86, for use in treating at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.

**88.** The pharmaceutical product according to claim 87, for use in treating breast cancer.

**89.** The pharmaceutical product according to claim 87, for use in treating stomach cancer.

**90.** The pharmaceutical product according to claim 87, for use in treating lung cancer.

**91.** The pharmaceutical product according to claim 90, wherein the lung cancer is non-small-cell lung cancer.

**92.** The pharmaceutical product according to any one of claim 70 to claim 91, wherein the pharmaceutical product is a pharmaceutical composition.

**93.** The antibody-drug conjugate according to claim 25, wherein the anti-HER3 antibody-drug conjugate is represented by the following formula:

[Formula 5]

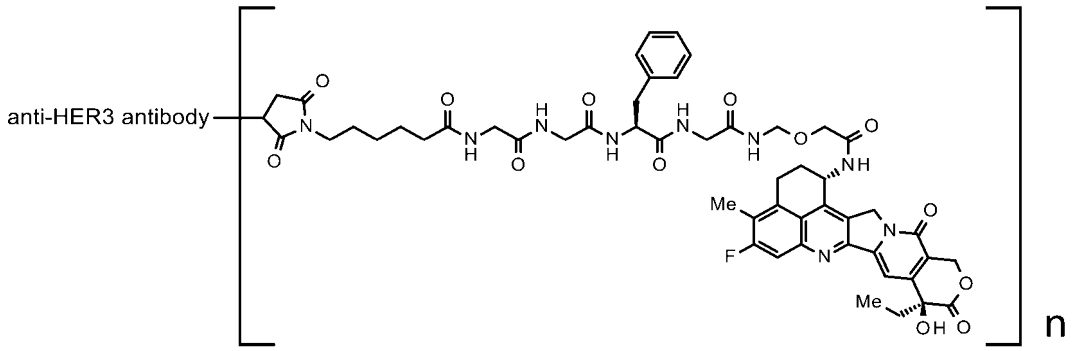

wherein 'anti-HER3 antibody' in the formula is an anti-HER3 antibody or a functional fragment thereof; a drug linker is conjugated to the antibody or functional fragment thereof via a thioether bond; and n represents drug-to-antibody ratio.

**94.** The antibody-drug conjugate according to claim 93, wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.

**95.** The antibody-drug conjugate according to claim 93, wherein the anti-HER3 antibody or functional fragment thereof comprises a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.

**96.** The antibody-drug conjugate according to claim 93, wherein the anti-HER3 antibody or functional fragment thereof comprises a heavy chain consisting of an amino acid sequence represented by SEQ ID NO: 1, and a light chain consisting of an amino acid sequence represented by SEQ ID NO: 2.

**97.** The antibody-drug conjugate according to claim 96, wherein the anti-HER3 antibody lacks a lysine residue at the carboxyl terminus of the heavy chain.

**98.** The antibody-drug conjugate according to any one of claims 93 to 97, wherein n is 2 to 8.

**99.** The antibody-drug conjugate according to any one of claims 93 to 97, wherein n is 3 to 8.

**100.**
The antibody-drug conjugate according to any one of claims 93 to 97, wherein n is 7 to 8.

**101.**
The antibody-drug conjugate according to any one of claims 93 to 97, wherein n is 7.5 to 8.

**102.**
The antibody-drug conjugate according to any one of claims 93 to 97, wherein n is 8.

**103.**

The antibody-drug conjugate according to any one of claims 93 to 102, wherein the anti-HER3 antibody-drug conjugate is patritumab deruxtecan.

**104.**
The antibody-drug conjugate according to any one of claims 93 to 103, wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

**105.**
The antibody-drug conjugate according to any one of claims 93 to 103, wherein the RASG12C inhibitor is sotorasib (AMG510), adagrasib (MRTX849), or a pharmaceutically acceptable salt thereof.

**106.**
The antibody-drug conjugate according to any one of claims 93 to 103, wherein the RASG12C inhibitor is sotorasib (AMG510), or a pharmaceutically acceptable salt thereof.

**107.**
The antibody-drug conjugate according to any one of claims 93 to 103, wherein the RASG12C inhibitor is adagrasib (MRTX849), or a pharmaceutically acceptable salt thereof.

**108.**
The antibody-drug conjugate according to any one of claims 93 to 107, wherein the antibody-drug conjugate and the RASG12C inhibitor are comprised in different formulations, and are administered at the same time or at different times.

**109.**
The antibody-drug conjugate according to any one of claims 93 to 108, wherein the cancer is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.

**110.**
The antibody-drug conjugate according to claim 109, wherein the cancer is breast cancer.

**111.** The antibody-drug conjugate according to claim 109, wherein the cancer is stomach cancer.

**112.**
The antibody-drug conjugate according to claim 109, wherein the cancer is lung cancer.

**113.**
The antibody-drug conjugate according to claim 112, wherein the lung cancer is non-small-cell lung cancer.

**114.**
The method of treatment according to claim 47, wherein the anti-HER3 antibody-drug conjugate is represented by the following formula:

[Formula 6]

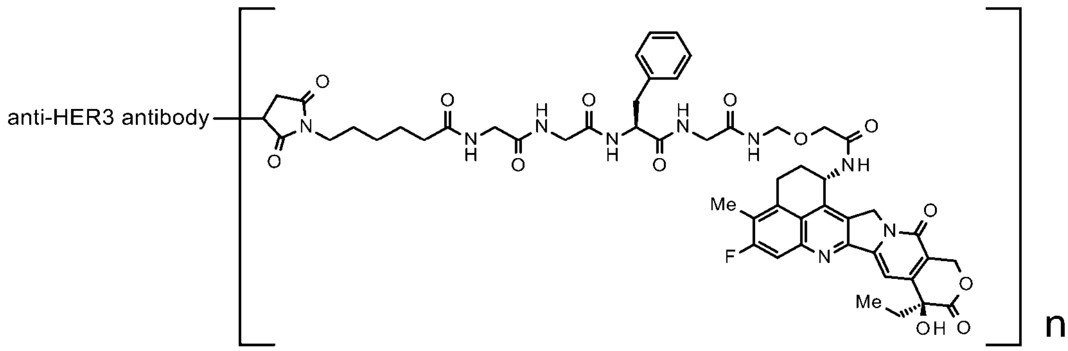

wherein 'anti-HER3 antibody' in the formula is an anti-HER3 antibody or a functional fragment thereof; a drug linker is conjugated to the antibody or functional fragment thereof via a thioether bond; and n represents drug-to-antibody ratio.

**115.**
The method of treatment according to claim 114, wherein the anti-HER3 antibody or functional fragment thereof comprises CDRH1 consisting of an amino acid sequence of amino acid residues 26 to 35 in SEQ ID NO: 1, CDRH2 consisting of an amino acid sequence of amino acid residues 50 to 65 in SEQ ID NO: 1, and CDRH3 consisting of an amino acid sequence of amino acid residues 98 to 106 in SEQ ID NO: 1; and CDRL1 consisting of an amino acid sequence of amino acid residues 24 to 39 in SEQ ID NO: 2, CDRL2 consisting of an amino acid sequence of amino acid residues 56 to 62 in SEQ ID NO: 2, and CDRL3 consisting of an amino acid sequence of amino acid residues 95 to 103 in SEQ ID NO: 2.

**116.**
The method of treatment according to claim 114, wherein the anti-HER3 antibody or functional fragment thereof is an antibody comprising a heavy chain variable region consisting of an amino acid sequence of amino acid residues 1 to 117 in SEQ ID NO: 1, and a light chain variable region consisting of an amino acid sequence of amino acid residues 1 to 113 in SEQ ID NO: 2.

**117.**
The method of treatment according to claim 114, wherein the anti-HER3 antibody or functional fragment thereof comprises a heavy chain consisting of an amino acid sequence represented by SEQ ID NO: 1, and a light chain consisting of an amino acid sequence represented by SEQ ID NO: 2.

**118.**
The method of treatment according to claim 117, wherein the anti-HER3 antibody lacks a lysine residue at the carboxyl terminus of the heavy chain.

**119.**
The method of treatment according to any one of claims 114 to 118, wherein n is 2 to 8.

**120.**
The method of treatment according to any one of claims 114 to 118, wherein n is 3 to 8.

**121.**
The method of treatment according to any one of claims 114 to 118, wherein n is 7 to 8.

**122.**
The method of treatment according to any one of claims 114 to 118, wherein n is 7.5 to 8.

**123.**
The method of treatment according to any one of claims 114 to 118, wherein n is 8.

**124.**
The method of treatment according to any one of claims 114 to 123, wherein the anti-HER3 antibody-drug conjugate is

patritumab deruxtecan.

**125.**
The method of treatment according to any one of claims 114 to 124, wherein the RASG12C inhibitor is a compound selected from the group consisting of LY3537982, AZD4625, sotorasib (AMG510), adagrasib (MRTX849), JDQ443, GDC-6036, BI1, 823, 911, D1553, and JNJ-74699157, or a pharmaceutically acceptable salt thereof.

**126.**
The method of treatment according to any one of claims 114 to 124, wherein the RASG12C inhibitor is sotorasib (AMG510), adagrasib (MRTX849), or a pharmaceutically acceptable salt thereof.

**127.**
The method of treatment according to any one of claims 114 to 124, wherein the RASG12C inhibitor is sotorasib (AMG510), or a pharmaceutically acceptable salt thereof.

**128.**
The method of treatment according to any one of claims 114 to 124, wherein the RASG12C inhibitor is adagrasib (MRTX849), or a pharmaceutically acceptable salt thereof.

**129.**
The method of treatment according to any one of claims 114 to 128, wherein the antibody-drug conjugate and the RASG12C inhibitor are comprised in different formulations, and are administered at the same time or at different times.

**130.**
The method of treatment according to any one of claims 114 to 129, wherein the disease is cancer.

**131.**
The method of treatment according to any one of claims 114 to 130, wherein the disease is at least one cancer selected from the group consisting of breast cancer, stomach cancer, colorectal cancer, lung cancer, esophageal cancer, head and neck cancer, gastroesophageal junction adenocarcinoma, biliary tract cancer, Paget disease, pancreatic cancer, ovarian cancer, bladder cancer, prostate cancer, uterine carcinosarcoma, gastrointestinal stromal tumor, kidney cancer, and sarcoma.

[Claim 132] The method of treatment according to claim 131, wherein the disease is breast cancer.
[Claim 133] The method of treatment according to claim 131, wherein the disease is stomach cancer.
[Claim 134] The method of treatment according to claim 131, wherein the disease is lung cancer.
[Claim 135] The method of treatment according to claim 134, wherein the lung cancer is non-small-cell lung cancer.

Fig. 3

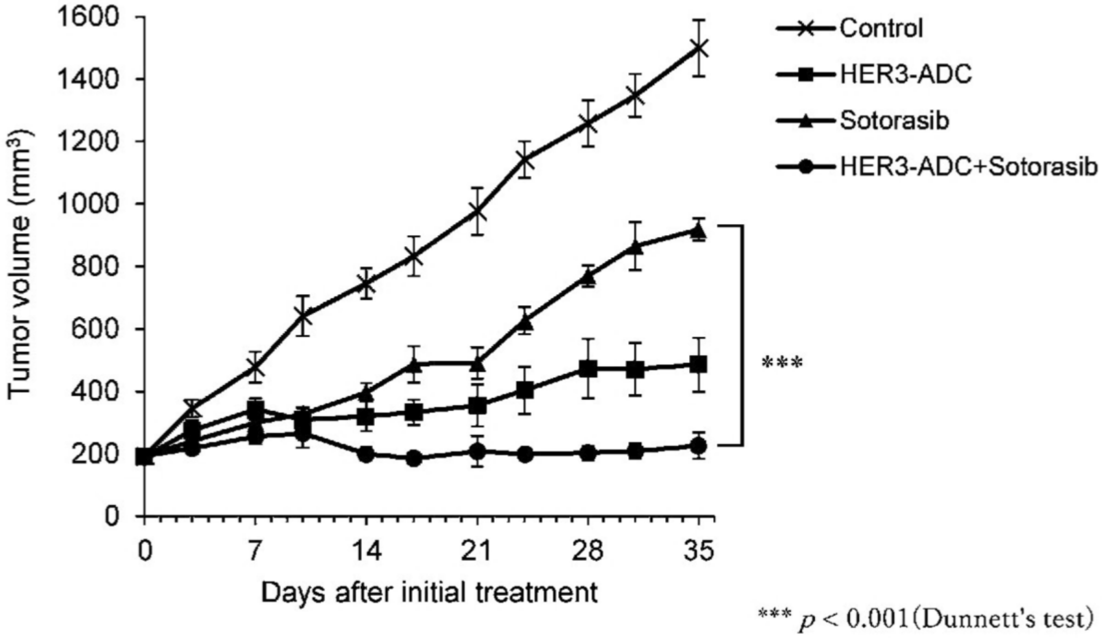
1600
1400
1200
1000
800
600
400
200
0
Tumor volume (mm³)
0
7
14
21
28
35
Days after initial treatment
Control
HER3-ADC
Sotorasib
HER3-ADC+Sotorasib
***
*** p < 0.001(Dunnett's test)

Fig. 4

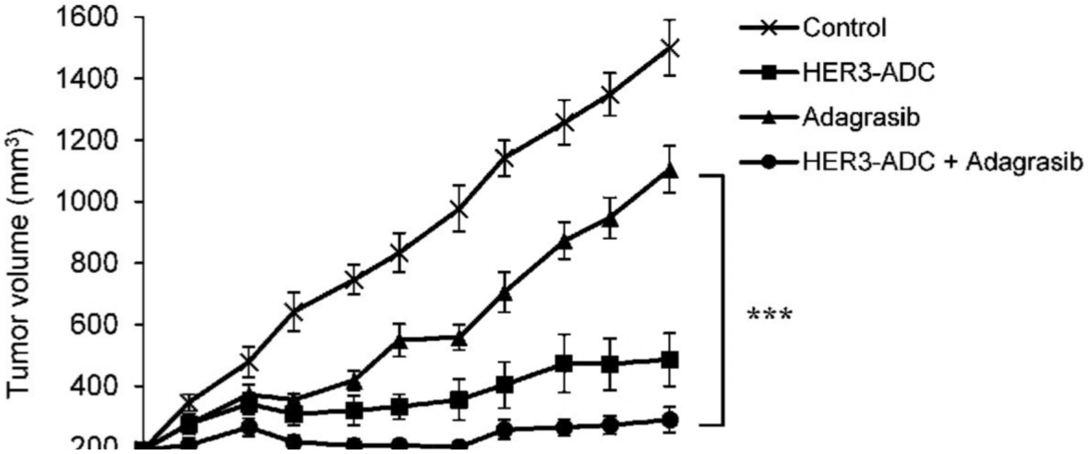
1600
1400
1200
1000
800
600
400
200
Tumor volume (mm³)
Control
HER3-ADC
Adagrasib
HER3-ADC + Adagrasib
***

Fig. 3

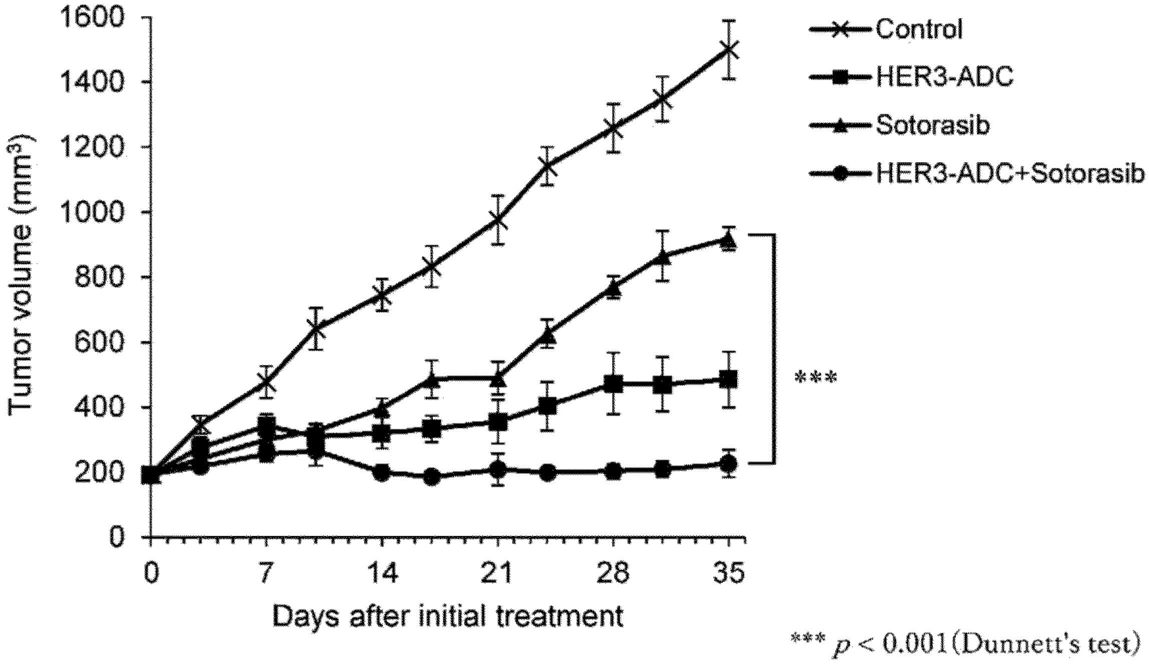


*** $p < 0.001$ (Dunnett's test)

Fig. 4

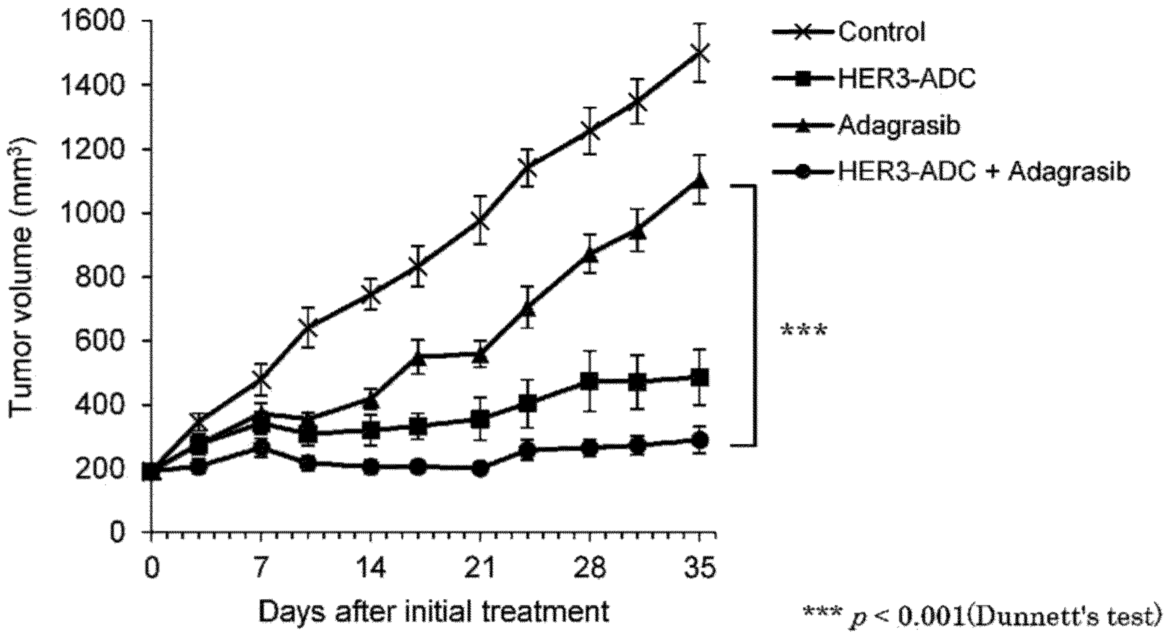


*** $p < 0.001$ (Dunnett's test)

## INTERNATIONAL SEARCH REPORT

International application No.
**PCT/JP2024/020005**

### A. CLASSIFICATION OF SUBJECT MATTER

***A61K 47/68***(2017.01)i; ***A61K 31/416***(2006.01)i; ***A61K 31/519***(2006.01)i; ***A61K 31/4745***(2006.01)i; ***A61K 39/395***(2006.01)i; ***A61K 45/00***(2006.01)i; ***A61K 47/65***(2017.01)i; ***A61P 35/00***(2006.01)i; ***A61P 43/00***(2006.01)i; ***C07K 16/28***(2006.01)i

FI: A61K47/68; A61K31/416; A61K31/4745; A61K31/519; A61K39/395 C; A61K39/395 D; A61K39/395 L; A61K39/395 N; A61K45/00 ZNA; A61P35/00; A61P43/00 121; C07K16/28; A61K47/65

According to International Patent Classification (IPC) or to both national classification and IPC

### B. FIELDS SEARCHED

Minimum documentation searched (classification system followed by classification symbols)

A61K47/00-47/69; A61K31/00-33/44; A61K39/00-39/44; A61K45/00-45/06; A61P35/00; A61P43/00; C07K16/00-16/46

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | JP 2017-503784 A (DAIICHI SANKYO COMPANY, LIMITED) 02 February 2017 (2017-02-02)<br>claims 1, 14, 24, 30-32, paragraphs [0072], [0088], [0091], [0190], [0191], [0197], [0250], [0256], [0286]-[0334] | 25-46, 93-113 |
| Y | | 1-135 |
| Y | WO 2022/269525 A1 (NOVARTIS AG) 29 December 2022 (2022-12-29)<br>claims 1, 2, p. 9, lines 6-8, p. 16, line 27 to p. 17, Line 20 | 1-135 |
| Y | ROBICHAUX, J. P. et al. Abstract P256: Pan-ErbB inhibition enhances activity of KRASG12C inhibitors in preclinical models of KRASG12C mutant cancers. Molecular Cancer Therapeutics. 2021, vol. 20, supplement 12, P256, ISSN 1538-8514<br>lines 16-33 | 1-135 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 July 2024** | **13 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No. **PCT/JP2024/020005**

**C. DOCUMENTS CONSIDERED TO BE RELEVANT**

| Category* | Citation of document, with indication, where appropriate, of the relevant passages | Relevant to claim No. |
|---|---|---|
| A | DESAI, O. et al. HER3- A key survival pathway and an emerging therapeutic target in metastatic colorectal cancer and pancreatic ductal adenocarcinoma. Oncotaget. 10 May 2023, vol. 14, pp. 439-443, ISSN 1949-2553 abstract, p. 440, left column, lines 35-39 | 1-135 |
| A | SOLANKI, H. S. et al. Cell Type-specific Adaptive Signaling Responses to KRASG12C Inhibition. Clinical Cancer Research. 2021, vol. 27, pp. 2533-2548, ISSN 1557-3265 abstract | 1-135 |

Form PCT/ISA/210 (second sheet) (July 2022)

**INTERNATIONAL SEARCH REPORT**

International application No.
**PCT/JP2024/020005**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/020005**

| Patent document cited in search report | Publication date (day/month/year) | Patent family member(s) | Publication date (day/month/year) |
|---|---|---|---|
| JP 2017-503784 A | 02 February 2017 | WO 2015/155998 A1<br>claims 1, 14, 24, 30-32, paragraphs [0072], [0088], [0091], [0190], [0191], [0197], [0250], [0286]-[0334] | |
| WO 2022/269525 A1 | 29 December 2022 | AU 2022299651 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020165732 A
- WO 2015155998 A
- WO 2013155223 A
- WO 2014143659 A
- WO 2014152588 A
- WO 2014160200 A
- WO 2015054572 A
- WO 2016044772 A
- WO 2016049524 A
- WO 2016164675 A
- WO 2016168540 A
- WO 2017058805 A
- WO 2017015562 A
- WO 2017058728 A
- WO 2017058768 A
- WO 2017058792 A
- WO 2017058807 A
- WO 2017058902 A
- WO 2017058915 A
- WO 2017087528 A
- WO 2017100546 A
- WO 2017201161 A
- WO 2018064510 A
- WO 2018068017 A
- WO 2018119183 A
- WO 2018217651 A
- WO 2018140512 A
- WO 2018140513 A
- WO 2018140514 A
- WO 2018140598 A
- WO 2018140599 A
- WO 2018140600 A
- WO 2018143315 A
- WO 2018206539 A
- WO 2018218070 A
- WO 2018218071 A
- WO 2019051291 A
- WO 2019099524 A
- WO 2019110751 A
- WO 2019141250 A
- WO 2019150305 A
- WO 2019155399 A
- WO 2019213516 A
- WO 2019213526 A
- WO 2019217307 A
- WO 2019215203 A
- WO 2019217691 A
- WO 2020178282 A
- WO 2021118877 A
- WO 2021245051 A
- WO 9007861 A
- US 5821337 A
- WO 9954342 A
- WO 0061739 A
- WO 0231140 A
- WO 2007077028 A
- WO 2011136911 A
- WO 2012022814 A
- WO 2010108127 A
- WO 2011047180 A
- WO 2014108484 A
- WO 2008100624 A
- WO 2013048883 A
- WO 2013063702 A
- US 6214345 B
- WO 2002083067 A
- WO 2003026577 A
- WO 2004054622 A
- WO 2005112919 A
- WO 2006135371 A
- WO 2007112193 A
- WO 2008033891 A
- WO 2009100194 A
- WO 2009134976 A
- WO 2009134977 A
- WO 2010093395 A
- WO 2011130613 A
- WO 2011130616 A
- WO 2013055993 A
- WO 2014057687 A
- WO 2014061277 A
- WO 2014107024 A
- WO 2014134457 A
- WO 2014145090 A
- WO 2012019024 A
- WO 2012064733 A
- US 20210353764 A
- WO 2020063676 A
- US 20240026028 A
- WO 2022078425 A
- WO 2023143365 A
- WO 2023041006 A
- WO 2023088382 A
- WO 2023125530 A
- WO 2023138635 A
- WO 2023143263 A
- WO 2023208216 A

- WO 2024077277 A
- WO 2024078449 A
- WO 2024083166 A
- WO 2024088388 A

**Non-patent literature cited in the description**


- **N.V. SERGINA et al.** *Nature*, 25 January 2007, vol. 445 (7126), 437-441
- **K YONESAKA et al.** *Oncogene*, 2016, vol. 35, 878-886
- **DUCRY, L. et al.** *Bioconjugate Chem.*, 2010, vol. 21, 5-13
- **ALLEY, S. C. et al.** *Current Opinion in Chemical Biology*, 2010, vol. 14, 529-537
- **DAMLE N. K.** *Expert Opin. Biol. Ther.*, 2004, vol. 4, 1445-1452
- **SENTER P. D. et al.** *Nature Biotechnology*, 2012, vol. 30, 631-637
- **HOWARD A. et al.** *J Clin Oncol*, vol. 29, 398-405
- *Cell Death and Differentiation*, 2008, vol. 15, 751-761
- *Molecular Biology of the Cell*, December 2004, vol. 15, 5268-5282
- *Bio Techniques*, January 2000, vol. 28, 162-165
- **KOHLER** ; **MILSTEIN**. *Nature*, 1975, vol. 256, 495-497
- Monoclonal Antibodies. Plenum Press, 1980, 365-567
- Proc. Natl. Acad. Sci. U. S. A.. 1984, vol. 81, 6851-6855
- Nature. 1986, vol. 321, 522-525
- **TOMIZUKA, K. et al.** *Nature Genetics*, 1997, vol. 16, 133-143
- **KUROIWA, Y. et al.** *Nucl. Acids Res.*, 1998, vol. 26, 3447-3448
- **YOSHIDA, H. et al.** Animal Cell Technology: Basic and Applied Aspects. Kluwer Academic Publishers, 1999, vol. 10, 69-73
- **TOMIZUKA, K. et al.** *Proc. Natl. Acad. Sci. USA*, 2000, vol. 97, 722-727
- **WORMSTONE, I. M. et al.** *Investigative Ophthalmology & Visual Science*, 2002, vol. 43 (7), 2301-2308
- **CARMEN, S. et al.** *Briefings in Functional Genomics and Proteomics*, 2002, vol. 1 (2), 189-203
- **SIRIWARDENA, D. et al.** *Ophthalmology*, 2002, vol. 109 (3), 427-431
- *Journal of Chromatography A*, 1995, vol. 705, 129-134
- *Analytical Biochemistry*, 2007, vol. 360, 75-83
- **OGITANI Y. et al.** *Clinical Cancer Research*, 29 March 2016, vol. 22 (20), 5097-5108
- **OGITANI Y. et al.** *Cancer Science*, 2016, vol. 107, 1039-1046
- **HERMANSON, G. T**. Bioconjugate Techniques. Academic Press, 1996, 56-136, 456-493